(19) Europäisches Patentamt
European Patent Office
Office européen des brevets

(11) EP 2 077 092 A2

(12) EUROPEAN PATENT APPLICATION

(43) Date of publication:
**08.07.2009 Bulletin 2009/28**

(51) Int Cl.:
***A61B 5/1473*** *(2006.01)*

(21) Application number: **07119527.5**

(22) Date of filing: **29.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Taipei Veterans General Hospital**
**Beitou District, 112 Taipei City (TW)**

(72) Inventors:
- **Chiou, Shih-Hwa**
  **112, Taipei City (TW)**
- **Tsai, Tung-Hu**
  **112, Taipei City (TW)**
- **Chang, Yuh-Lih**
  **112, Taipei City (TW)**
- **Yen, Chih-Ju**
  **112, Taipei City (TW)**

(74) Representative: **Lang, Christian et al**
**LangRaible GbR**
**Patent- und Rechtsanwälte**
**Herzog-Wilhelm-Straße 22**
**80331 München (DE)**

(54) **System and Methods for Screening or Analyzing Targets**

(57) The present invention relates to the system and methods for screening or analyzing targets.

FIGURE 1
(A)

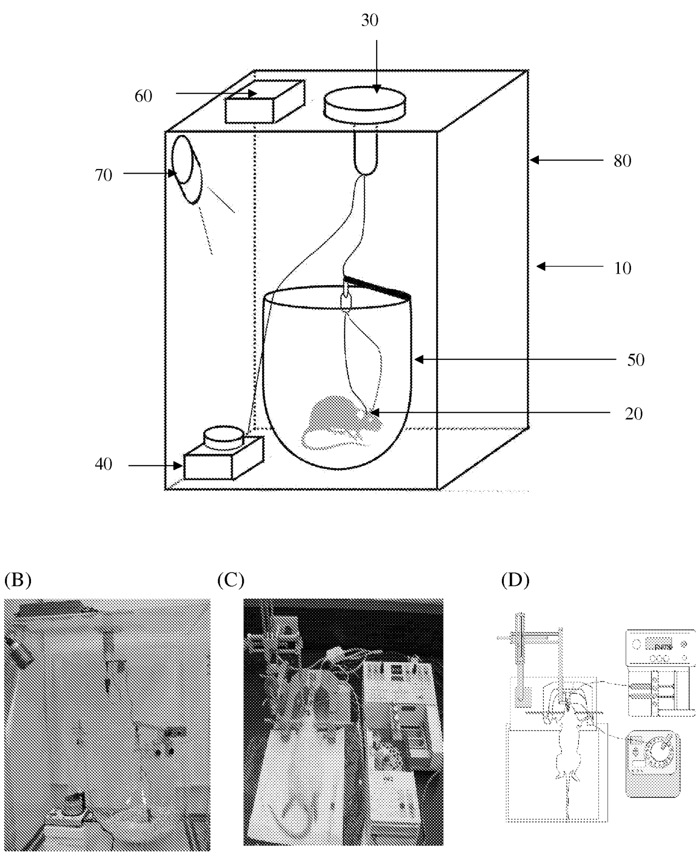


EP 2 077 092 A2

## Description

## FIELD OF THE INVENTION

The present invention relates to system and methods for screening or analyzing targets. In particular, the invention relates to the system and methods for screening or analyzing drugs for treating or preventing neural-related, diabetic, or other diseases.

## DESCRIPTION OF PRIOR ART

Various drug screening methods and devices have been disclosed in literatures or patents, such as nucleic acid detection, fluorescence detection, cell viability assay, complementation assay.

Microdialysis is one of the most widely used techniques for in vivo or in vitro sampling of the chemical substances in intracellular and extracellular fluids of animal tissues, or cultured cells.

Microdialysis works by slowly pumping a solution (the "dialysate") through the microdialysis probe. Molecules in the tissues diffuse into the dialysate as it is pumped through the probe; the dialysate is then collected and analyzed to determine the identities and concentrations of molecules that were in the extracellular fluid (ECF). The concentration in the dialysate of any given substance will normally be much lower than the concentration present in the extracellular fluid, especially for substances of relatively high molecular weight. Typically the concentration of a peptide collected by microdialysis will be just 5-10% of the original concentration. This depends on the charge and size of the molecule in question as well as the dialysis speed. Different techniques can be used to assay the dialysis time needed to attain steady state including radioactive labeled probes. Analysis of the fluid can occur in a laboratory or at a patient's bedside, if microdialysis is being used in a clinical context.

Some microdialysis methods and devices are disclosed in No. 6,591,126, U.S. Pat. No. 6,463,312, U.S. Pat. No. 6,091,976, U.S. Pat. No. 6,030,358, U.S. Pat. No. 5,741,284, U.S. Pat. No. 5,735,832, U.S. Pat. No. 5,706,806, U.S. Pat and No. 5,607,390, all of which are herein incorporated by reference in their entirety.

A variety of analytical methods, roughly sorted into immune and non-immune methods, had been applied for insulin detection. The former one had been used for detecting insulin in vivo early from 1950s, which mainly included radioimmunoassay (RIA), enzyme immunoassay (EIA), and luminescent immunoassay (LIA). The non-immune methods, especially high performance liquid chromatography (HPLC) and capillary electrophoresis (CE), had been widely used for insulin detection in vivo and in vitro. As known, non-immune method for insulin detection had insufficient sensitivity compared with immune method because of the interferences from the sample matrix. However, HPLC could be a rapid and accuracy method, if proper pretreatment procedure was applied for reverse-phase HPLC, a typical analytical method for quantifying peptide and protein in liquid drug, owned the high resolution and easy automation to meet the sample test requests.

In present HPLC system, the chromolith C18 reversed-phase column that has been used to separate several drugs and pollutant compounds. However, up to now, it has not been applied in determining insulin; measurement of extracellular insulin in culture medium using conventional LC-UV has been difficult and challenging. For example, the extremely low concentration of insulin in medium requires a very low analytical detection limit for successful measurement and the medium samples require time-consuming pretreatments or complicated extraction prior to analysis by LC-UV assays, which results in sample losses and increases in the amount of time necessary to complete experiments. In additional, the efficacy of candidate drugs regulate insulin related gene expression has not been investigated.

In order to develop more rapid, efficient/quantitative, real-time, and auto-sampling disease treatments, new drug screening methods and devices are still investigated and improved.

## BRIEF DESCRIPTION OF THE DRAWINGS

Figure 1 shows (A) and (B) which are directed to the microdialysis system of the present invention for use in *in vivo* analysis and animal models. In the figure 1 (A), 10 is the system of the present invention; 20 is a microdialysis probe; 30 is autosampling pumping; 40 is a auto-collector; 50 is a rotatory (free moving) housing; 60 is a pump; 70 is a light and 80 is a transparent box. The animal behavior and other drug-related response can be continuously/long-term observed or recorded by observers or CCD camera. (C) and (D) the previous microdialysis system (fixation type) for use in *in vivo* analysis.

Figure 2 shows the section of hippocampus, and arrow indicates the probe insert position.

Figure 3 shows another embodiment of a microdialysis system for in vitro analysis. In the figure, A is guide cannulae; B is dialysis membrane (30 mm in length); C is Culture medium; D is Petri dish cover; E is Petri dish body; F is PE-10; G is running foot tubing and H is silica tubing.

Figure 4 shows one preferred embodiment of the present invention for simultaneously monitoring the differentiation process (the number of neurite, neurite length, and primary dendrites) of neural stem cells under the observations of CCD confocal microscope and dopamine (DA) and serotonin (5-HT) concentrations by microdialysis with HPLC-ED. In the figure, 10 is the system of the present invention; 20 is a $CO_2$ incubator; 30 is a pump; 40 is a collector; 50 is an apparatus for performing polymerase chain reaction; 60 is a HPLC-ED; 70 is a device for performing ELISA; 80 is a camera and 90 is a computer.

Figure 5 shows evaluation of the differentiation process of neural stem cell by *in vitro* green fluorescent protein gene (GFP) imaging system.

Figure 6 shows neural stem cells (NSCs) differentiated into functional neuron by *in vitro* GFP imaging system.

Figure 7 shows MRI scans acquired in axial (A) coronal (B) planes. Microscopic Images (C) Dissecting microscope (D) *In vivo* GFP imaging (E) *Ex vivo* GFP imaging. Digital Microscopic Images (F) Microscopic (G) green fluorescence (H) F and G merged. In the figure, 10 is a microdialysis probe; 30 is a pump; 40 is a collector; 70 is a HPLC-ED; 110 is a rat brain and 115 is transplanted cells and/or tissues.

Figure 8 shows (A) standard dopamine (DA) and serotonin (5-HT) at each concentration of 50ng/ml by HPLC-ED. (B) dialysate sample collected from non-induced PC-12 cells medium with basal concentration of 6.04ng/ml and 0.18ng/ml for DA and 5-HT. (C) dialysate sample containing 50.06ng/ml DA and 2.94ng/ml 5-HT collected after induction with NGF of PC-12 cells analyzed by HPLC-ED.

Figure 9. The 5' -414 to -133 bp region of the c-FLIP promoter responds to fluoxetine activation in fluoxetine-treated neural stem cells (NSCs). (A) A schematic diagram of the deletion mutants of c-FLIP promoter. (B) NSCs were transfected with vector only (PGL3: control; lanes 1 and 2), full-length of c-FLP promoter (lanes 3 and 4) or deleted mutants of the c-FLIP promoter (lanes 5 and 6: -696 to +150 bp; lanes 7 and 8: -414 to +150 bp; lanes 9 and 10: -132 to +150 bp), followed by luciferase assays and Western analyses. FL: fluoxetine 20 μm added (lane 2, 4, 6, 8, and 10), control: no FL (1, 3, 5, 7, and 9). (#: $p<0.05$, compared to full-length, -696 to +150 bp, and -414 to +150 bp). (C) Detection of protein levels of c-FLIP induced by FL in different doses. (D) FL-treated NSCs on Day 5 were collected and double stained of c-FLIP (green) and MAP2 (red). Bar: 20 μm. (E) 10, 50, and 100μM of the PI3 kinase inhibitor LY294002 added into FL-treated NSCs for 1h (lane 5) fails to induce the protein level of c-$FLIP_L$. Data shown here are the mean ± SE of three experiments.

Figure 10 shows a preferred embodiment of the microdialysis system with HPLC assay for in vitro analysis. (A) This device can be applied for detecting the secreting products (ex. Insulin) for floating (suspension) cells or 3-D tissues. (B) The illustrated figure for this device, A is PE-10; B is epoxy; C is PE-60; D is a cover; E is silica tubing; F is dialysis membrane; and G is cells (or tissues). (C) In the figure, 10 is a microdialysis probe; 30 is an autopump; 40 is an autocollector; 50 is an apparatus for performing polymerase chain reaction; 60 is a HPLC-ED and 70 is a HPLC-ED. (D) Using this device and detection system, the insulin levels (0.1~20 μg/ml) form six samples including placenta-derived multipotent stem cells (PDMSCs) were analyzed by HPLC and chromatography.

Figure 11 illustrates the application fields of the present invention.

Figure 12 illustrates the use of *in vitro* microdialysis of the present invention in *in vitro* screening model of stem cell for studying drug mechanism, new candidate gene and new drugs.

Figure 13 shows (A) a typical chromatogram of a standard mixture containing cefoperazone (50 μg/ml); (B) the blank sample, the chromatographic conditions revealed no biological substances that would interfere significantly with the determination of cefoperazone; and (C) a chromatogram of microdialysate from rat biliary duct.

Figure 14 shows isolation and cultivation of NSCs from the hippocampal region of adult SD rats. (A) NSCs aggregated and formed neurospheres under serum-free medium culture. Detection of the signals of (B) Nestin protein (red color), (C) DAPI (blue color), and (D) Nestin/DAPI merged imaging. With an added 2% FBS, the neurosphere is then attached to the culture plate and gradually differentiated into (E) neural-like cells. (F) The MAP-2 positive (green color) neurons were detected in the differentiated NSCs (A-F was performed by three separate experiments; Bar: 40 μm). By using MTT assay, the cell viability and survival of NSCs in the different IM-treated concentration were evaluated. NSCs were treated with 0, 1, 2, 3, 4, 5, 10, 20, 30, 40, 50, 100, and 200 μM imipramine (IM) for (G) 3, (H) 5, and (I) 7 days. The data (the mean±S.E.M. of six separate experiments) are expressed as percentages of the control value (non IM-treated).

Figure 15 shows detection of mRNA expression in imipramine (IM)-treated NSCs by quantitative RT-PCR. By using a real-time RT-PCR method, the mRNA expression levels of (A) BDNF, (B) Bcl-2, (C) Bax, (D) Bad, and (E) MAP-2 genes were measured in IM-treated and non-IM-treated NSCs on different culture days (Day 3, Day 5 and Day 7). The data shown here are the mean ± S.E.M. of three experiments;*$p<0.05$ and **$p<0.01$, IM-treated NSCs as compared to the same days of non-IM-treated NSCs. (F) The mRNA expression levels of Bcl-2 in NSCs treated with different concentrations (1, 3, 5, 10 μM) of IM in 7 days culture. The data shown here are the mean ± S.E.M. of three experiments; *$p<0.05$ and **$p<0.01$, as compared to the Day 7 non-IM-treated NSCs.

Figure 16 shows detection of Bc1-2 expressions in IM-treated NSCs using western blot and immunochemistry methods. (A) The expression levels of Bcl-2 protein in Day 3, Day 5, and Day 7 IM-treated NSCs were significantly higher than those in Day 3 non-IM-treated NSCs (Control; *$p<0.05$ and **$p<0.01$). The Bc1-2 level in Day 7 IM-treated NSCs was significantly inhibited by the transfection of Bcl-2 siRNA (Bcl-2 siRNA (D7+IM); #$p<0.05$). The data shown here are the mean ± S.E.M. of three experiments. (B) By immunocytochemistry, the protein levels of Bcl-2 (Arrows; red color) were significantly increased and localized in the cytoplasm of Day 7 IM-treated NSCs as compared to Day 7 non-IM-treated NSCs. The expression of the Bc1-2 protein induced by IM treatment was effectively inhibited by the transfection of Bcl-2 siRNA in IM-treated NSCs as compared to siRNA control (B was performed by three separate experiments; Bar: 10 μm). (C) The protein level of Bcl-2 was up-regulated by IM-only or BDNF-only treatment. The expression of the Bcl-2 protein induced by IM was inhibited by the treatment with BDNF siRNA, U0126 (MAPK-pathway specific inhibitor), and Bcl-2 siRNA.

Figure 17 shows IM treatment increasing the cell survival rate in LPS-treated NSCs. (A) IM and BDNF treatment protect against LPS-induced cell death (Bar: 50 μm). (B) The cell viabilities and cell survival rate in NSCs were significantly decreased with LPS treatment (†$p<0.05$, LPS vs. control; Control: medium only; LPS: NSCs treated with LPS 100 ng/ml). The cell survival rate in LPS-treated NSCs were significantly increased with IM or BDNF treatment (*$p<0.05$, BDNF+LPS vs. LPS, or IM+LPS vs. LPS). The cell survival rate of LPS-treated NSCs with IM treatment was significantly decreased in cells treated with BDNF siRNA, U0126, and Bcl-2 siRNA, respectively (#$p<0.05$, IM+LPS+BDNF siRNA vs. IM+LPS, IM+LPS+U0126 vs. IM+LPS, or IM+LPS+Bcl-2 siRNA vs. IM+LPS). The cell survival rate of LPS-treated NSCs with IM treatment was significantly decreased in cells treated with BDNF siRNA or Bcl-2 siRNA than in those with siRNA control (+$p<0.05$, IM+LPS+BDNF siRNA vs. IM+LPS+siRNA control, IM+LPS+Bcl-2 siRNA vs. IM+LPS+siRNA control). The data shown here are the mean ± S.E.M. of three experiments. (C) By western blotting, the protein expression level of Bcl-2 in 3 μM IM- LPS-treated NSC group was found to be higher than that in the groups of 1, 2 μM IM-LPS-treated NSCs, the only LPS-treated NSCs, and the control group.

Figure 18 shows evaluation of LPS-induced apoptotic activity in NSCs treated with and without imipramine by caspase 8 and 3 ELISA test and TUNEL assay. (A) To investigate the neuroprotective ability of IM treatment in LPS-treated NSCs, the apoptotic activities of caspase 8 and (B) caspase 3 induced by LPS in IM or non-IM pretreated NSCs were evaluated by ELISA assay. (The ratio of non-IM treated NSCs was defined as 0.1, and the relative activities of caspase 8 and 3 in the differently treated groups were compared to non-IM treated NSCs.) (C) The severity of DNA fragmentation induced by LPS in the IM and non-IM pretreated NSCs was analyzed by TUNEL assay. Apoptotic activities were significant increased with LPS treatment (†$p<0.05$, LPS vs. control; Control: medium only; LPS: NSCs treated with LPS 100 ng/ml). Compared to the only LPS-treated NSCs, IM or BDNF treatment significantly inhibited apoptotic activities in LPS-treated NSCs (*$p<0.05$, BDNF+LPS vs. LPS, or IM+LPS vs. LPS). Compared to the only LPS-treated NSCs with IM, the treatment with BDNF siRNA, U0126 (MAPK inhibitor; 40 μM), or Bcl-2 siRNA can significantly increase apoptotic activities in LPS-treated NSCs with IM (#$p<0.05$, IM+LPS+BDNF siRNA vs. IM+LPS, IM+LPS+U0126 vs. IM+LPS, or IM+LPS+Bcl-2 siRNA vs. IM+LPS). Compared to the IM and LPS-treated NSCs with siRNA control, the treatment with BDNF siRNA or Bcl-2 siRNA can significantly increase apoptotic activities. (‡$p<0.05$, IM+LPS+BDNF siRNA vs. IM+LPS+siRNA control, IM+LPS+Bcl-2 siRNA vs. IM+LPS+siRNA control). The data shown here are the mean ± S.E.M. of three experiments.

Figure 19 shows detection of the production of IL-1β, IL-6, and TNF-α levels in LPS-treated NSCs by ELISA tests. To investigate the LPS-induced pro-inflammatory reaction and anti-inflammatory effects of IM treatment in LPS-treated NSCs for 7 days, the concentration of pro-inflammatory cytokines was detected by ELISA. The concentrations of the (A) IL-6, (B) IL-1β, and (C) TNF-α induced by LPS in the IM-treated NSCs with the different treatment groups. The production of IL-6, IL-1β, and TNF-α was significant increased with LPS treatment (†$p<0.05$, LPS vs. control; Control: medium only; LPS: NSCs treated with LPS 100 ng/ml). Compared to the only LPS-treated NSCs, IM or BDNF treatment significantly inhibited the production of IL-6, IL-1β, and TNF-α in LPS-treated NSCs (*$p<0.05$, BDNF+LPS vs. LPS, or IM+LPS vs. LPS). Compared to the only LPS-treated NSCs with IM, the treatment with BDNF siRNA, U0126 (MAPK inhibitor; 40 μM), or Bcl-2 siRNA can significantly increase the levels of IL-6, IL-1β, and TNF-α in the LPS-treated NSCs with IM (#: $p<0.05$, IM+LPS+BDNF siRNA vs. IM+LPS, IM+LPS+U0126 vs. IM+LPS, or IM+LPS+Bcl-2 siRNA vs. IM+LPS). Compared to the IM and LPS-treated NSCs with siRNA control, the treatment with BDNF siRNA or Bcl-2 siRNA can significantly increase the levels of IL-6, IL-1β, and TNF-α (‡$p<0.05$, IM+LPS+BDNF siRNA vs. IM+LPS+siRNA control, IM+LPS+Bcl-2 siRNA vs. IM+LPS+siRNA control). Data shown here are the mean ± S.E.M. of three experiments.

Figure 20 shows imipramine (IM) promotes NSCs differentiation into serotoninergic neurons. Detection of the protein expression of (A) MAP2 (green fluorescence; A, C, G, and I), (B) DAPI (blue fluorescence; B, C, E, F, H, and I), (D) 5-HT (red fluorescence; D, F, G, and I), and merged imaging (C, F, G, and I) in the same differentiating cells derived from IM-treated NSCs by using triple-staining immnunofluorescent assay (Bar: 5 μm). The percentages of (J) MAP2-positive neurons, (K) 5-HT-positive cells, and (L) both of the 5-HT /MAP2-positive cells in the IM-treated and non-IM-treated NSCs were measured by immunofluorescent signals. (J-L) The percentages of both MAP2-positive and 5-HT-positive cells in IM-treated NSCs were significantly higher than those in non-IM-treated NSCs (*$p<0.05$, NSC + IM vs. non-IM-treated (control) group). The induction rate of 5-HT-positive cells or MAP2-positive cells in IM-treated NSCs was significantly inhibited by BDNF siRNA, U0126, or Bcl-2 siRNA. (#: $p<0.05$, IM+ BDNF siRNA vs. IM, IM +U0126 vs. IM, or IM +Bcl-2 siRNA vs. IM). Compared to the IM-treated NSCs with siRNA control, the treatment with BDNF siRNA or Bcl-2 siRNA can significantly decrease the induction rate of 5-HT-positive or MAP2-positive cells (‡$p<0.05$, IM+BDNF siRNA vs. IM+siRNA control, IM+Bcl-2 siRNA vs. IM+siRNA control). The data shown here are mean ± S.E.M. of three experiments.

Figure 21 shows detection of 5-HT levels in the culture medium of IM-treated NSCs by microdialysis with HPLC-ECD. (A) By using microdialysis with HPLC-ECD, the 5-HT levels were continuously monitored in the culture medium of 24, 48, 72 and 96 hours in each groups. The concentration of 5-HT is higher in IM-treated NSCs compared to non-IM-treated NSCs. The levels of 5-HT induced by IM treatment was effectively inhibited by BDNF siRNA, U0126, or Bcl-2 siRNA. (B) The levels of 5-HT on Day 7 and Day 14 in IM-treated NSCs were significantly higher than those in non-IM-treated NSCs (*$p<0.05$, NSC + IM vs. non-IM-treated (control) group). The levels of 5-HT on Day 7 and Day 14 were significantly inhibited by BDNF siRNA, U0126, or Bcl-2 siRNA. (#: $p<0.05$, IM+ BDNF siRNA vs. IM, IM +U0126 vs. IM, or IM +Bc1-2 siRNA vs. IM). Compared to the IM-treated NSCs with siRNA control, the treatment with BDNF siRNA or Bcl-2 siRNA can significantly decrease the levels of 5-HT on Day 7 and Day 14 (‡$p<0.05$, IM+BDNF siRNA vs. IM+siRNA control, IM+Bc1-2 siRNA vs. IM+siRNA control).

Figure 22 shows detection of gene expressions and protein in 3D spheroid-bodies placenta-derived multipotent stem cells (SB-PDMSCs). (A) Detection of mRNA expressions by real-time RT-PCR. The mRNA expression levels of Sox17, Foxa2, Pdx1, insulin, glucagon, and somatostatin were activated in SB-PDMSCs in the four week culture (*$p<0.05$). (B) Photography of 3D SB-PDMSCs. (C) By immunofluorescent staining, insulin (red) was found to be highly expressed in the aggregated SB-PDMSCs. (D) Both of signals for insulin (red) and glucagon (green) co-expressed in SB-PDMSCs (bar: 50 μm). (E) The percentages of insulin-positive cells in SB-PDMSCs and PDMSCs (*$p<0.05$).

Figure 23 shows employment of SCID mice with STZ-pretreatment to examine the normoglycemia restoration of differentiated Placenta-derived multipotent stem cells (PDMSCs) in xenotransplantation. (A) PDMSCs were implanted into the subcapsular space of the left kidney (arrow: xenotransplanted graft-derived from PDMSCs). (B) The histological study revealed that transplanted PDMSCs formed solid tissue in the subrenal site. (C) The immunfluorescent tests confirmed that insulin (red)- and glucagon (green)-positive cells were detected in PDMSCs -derived tissues (bar: 50 μm). (D) The blood glucose levels of PDMSCs implanted group were significantly lower than those of undifferentiated PDMSCs and STZ-control groups ($p<0.05$).

## SUMMARY OF THE INVENTION

The present invention relates to a microdialysis system comprising: (i) a microdialysis apparatus including (a) a microdialysis probe formed to be inserted in a tissue or a transplanted tissue of an animal, the probe defining a probe channel having a perfusate inlet and a dialysate outlet and including a dialysis membrane, the dialysis membrane being formed to permit diffusion of fluid into the channel and to separate the probe channel from the surrounding tissue, (b) an input tube connected to the perfusate inlet; (c) an output tube connected to the dialysate outlet; and (ii) an assistant device for obtaining the dialysate, which is (d) a rotatory or free moving housing for placing the animal or monitoring *in vivo* treatment response and animal behavior or (e) a container for cultivating a cell medium.

The present invention also relates to an *in vivo* method for screening or analyzing a target comprising: (a) implanting a microdialysis probe into a tissue of a free-moving animal; (b) placing the animal into a rotatory or free moving housing; (c) adding the target along with a perfusate; and (d) determining the efficacy of the target by screening or analyzing a dialysate.

The present invention further relates to an *in vitro* method for screening or analyzing a target comprising: (a) cultivating a cell to a medium in a container; (b) inserting a microdialysis probe into the cell medium; (c) adding the target along with a perfusate; and (d) determining the efficacy of the target by screening or analyzing a dialysate.

## DETAILED DESCRIPTION OF THE INVENTION

The present invention relates to a microdialysis system comprising: (i) a microdialysis apparatus including (a) a microdialysis probe formed to be inserted in a tissue or a transplanted tissue of an animal, the probe defining a probe channel having a perfusate inlet and a dialysate outlet and including a dialysis membrane, the dialysis membrane being formed to permit diffusion of fluid into the channel and to separate the probe channel from the surrounding tissue, (b) an input tube connected to the perfusate inlet; (c) an output tube connected to the dialysate outlet; and (ii) an assistant device for obtaining the dialysate, which is (d) a rotatory or free moving housing for placing the animal or monitoring *in vivo* treatment response and animal behavior or (e) a container for cultivating a cell medium.

The term "tissue" used herein is not limited but includes a general tissue derived from in vivo, ex vivo or transplanted tissue or the growth of cells (such as stem cells). In a preferred embodiment, the tissue is brain, heart, liver, bile duct, pancreas, stomach, intestine or kidney. In a preferred embodiment, the cells are neural stem cell, mesenchymal stem cell, or stem cell.

In the system of the present invention, the input tube is linked to a continuous source of perfusate. For example, the continuous source of perfusate can be made by pump system. In a preferred embodiment, the autosampling system includes time-setting of autopump and/or autocollector.

In the system of the present invention, the output tube is linked to an analyzing device by a automatic sampling including time-setting of autopump and/or autocollector. The analyzing device is not limited but includes a high performance liquid chromatography system, an *in vivo or in vitro* green fluorescent protein gene (GFP) imaging device for fluorescent or gene-targeting guidance, an apparatus for analyzing gene-related or cytokine function, etc. In a preferred embodiment, the GFP imaging is expressed on a transplanted hippocampus, ventricle, or intraocular cavity by a transplanted cell with GFP or gene-targeting guidance.

In a preferred embodiment, the assistant device for obtaining the dialysate is a rotatory or free moving housing which is applied to (a) *in vivo* analysis for a free-moving animal, (b) drug screening or testing, (c) multiple time point or real time monitor, (d) automatic sampling including time-setting of autopump and/or autocollector; or (e) observation of an animal mode or behavior improvement treated by a drug.

In a preferred embodiment, the housing can be applied to observe a mice model for use in assaying a neurodegenerative disease (such as Alzheimer's-like, Parkinson's-like, emotional diseases-like, epilepsy-like, or inflammatory-related animal models) or identify the efficacy of a drug for treating neurodegenerative disease based on the behavior improvement of the mice after administering the drug.

In a more preferred embodiment, the housing further comprising a light to control or interrupt day and night duration.

In a preferred embodiment, the assistant device for obtaining the dialysate is the cultivating container which could be applied to (a) *in vitro* analysis, (b) drug screening or testing, (c) multiple time point or real time monitoring or (d) automatic sampling including time-setting of autopump and/or autocollector. In a more preferred embodiment, the container is in a disk form and the cell is a neuronal cell or attached cell. In a further preferred embodiment, the container has a transparent cover for observing inner space, cell morphological change or cell differentiation-regeneration processes and is positioned in an anaerobic enviroment (such as $CO_2$ incubator). Thus, the cells in the container under anaerobic condition could provide continuously, long-term (one to weeks), and real-time monitor the proliferation and differentiation process of cells or stem cells. In the present invention, it demonstrated that neural stem cells could differentiate into mature neurons, including dopamine-positive neurons, or serotonin (5-HT)-positive neurons.

In a preferred embodiment, the container could be in a cone form on the bottom of the container and the cell is a suspending cell, beta-islet cell, insulin-producing cell or hepatic cell. In the present invention, it demonstrated that the soluble-form insulin or other cytokines released from insulin-positive cells or tissues could be functionally detected by this new device.

The present invention also relates to an *in vivo* method for screening or analyzing a target comprising: (a) implanting a microdialysis probe into a tissue of a free-moving animal; (b) placing the animal into a rotatory or free moving housing; (c) adding the target along with a perfusate; and (d) determining the efficacy of the target by screening or analyzing a dialysate.

In a preferred embodiment, the method of the present invention further comprises transplantation of a cell expressing fluorescence on tissue to facilitate implanting a microdialysis probe into a position exhibiting fluorescence.

The term "target" used herein is not limited but includes a drug, peptide, polypeptide, compound, small molecule, extract etc. In particular, the target is one for treating or preventing neural or pancreatic diseases. In general, the target is identified or determined by cell or tissue normal function. In a preferred embodiment, the target can be determined by neurotransmitters (such as dopamine, serotonin, etc), hormone (such as insulin), growth factors or cytokines.

The present invention also relates to an *in vitro* method for screening or analyzing a target comprising: (a) cultivating a cell to a medium in a container; (b) inserting a microdialysis probe into the cell medium; (c) adding the target along with a perfusate; and (d) determining the efficacy of the target by screening or analyzing a dialysate. In a preferred embodiment, the cell is neural stem cell, beta-islet cell or insulin-producing cell.

## EXAMPLE

The examples below are non-limiting and are merely representative of various aspects and features of the present invention.

### Example 1: Microdialysis samples of freely moving animals

In this example, isocratic separation of pefloxacin from brain dialysate using a reversed- phase C18 column (150mm×4.6 mm; i.d. = 5 μm; Cosmosil, Kyoto, Japan) has been achieved within 10 min. The mobile phase consisted of citrate buffer (pH 5.0), acetonitrile and triethylamine (83:17:0.1, v/v/v). For this, one liter of citrate buffer (25 mM) was prepared using 25mM of sodium acetate and 25mM of citric acid dissolved in triple distilled HPLC grade water and buffered to pH 5.0 using orthorphosphoric acid. This mobile phase was filtered with a 0.45 μm Millipore membrane prior

to being used for elution. The chromatographic pump flow rate was set at 1 ml/min. The excitation and emission wavelengths for optimal fluorescence response for pefloxacin were determined to be 330 and 440 nm, respectively.

To investigate the fast releasing events of neurotransmitter release mechanisms, highly temporal resolution had been successfully improved in brain microdialysis for the measurement of neurochemicals. Improving temporal resolution for microdialysis sampling must overcome challenges such as lower sample volume, sensitive detection and fast collection. For brain microdialysis, the rat was mounted on a stereotaxic frame and perfused with Ringer's solution. After being washed with Ringer's solution at a flow-rate of 2 ml/min, the microdialysis probe was implanted in the right striatum according to the Paxinos and Watson atlas. The positions of the probes were verified by standard histological procedure at the end of experiments. The sampling device was perfused with the blank perfusate by a micropump (CMA-400). Microdialysates were introduced by an microfraction collector (CMA-142) and analyzed by HPLC. The equipped microfraction collector was used to collect microdialysates and then was assayed by HPLC system.

As shown in Figure 1, the sensitive and quantitative liquid chromatography method analyzed the extracellular concentrations of neurotransmitter in brain microdialysis samples of freely moving animals. Following induction of anesthesia, animals were secured in a stereotaxic frame with ear and incisor bars. Anesthesia was maintained by continuous administration while a microdialysis guide cannula (CMA Microdialysis, Sweden) was implanted above the dorsal hippocampus (AP: .4.3mm ML: .2.6mm DV: .2.1 mm). The guide cannula was secured to the skull using dental acrylic and two small stainless-steel screws. Following surgery, animals were individually housed in cages, with free access to food and water. The following day rats were used in microdialysis experiments. Microdialysis probes were perfused with artificial cerebrospinal fluid prior to insertion in the guide cannula. The microdialysis probe was then inserted into the dorsal hippocampus via the guide cannula. A stabilization period was allowed following probe insertion before dialysate sampling was initiated.

The sampling device was perfused with the blank perfusate by a micropump (CMA-400). Microdialysates were introduced by an microfraction collector (CMA-142) and analyzed by HPLC. The equipped microfraction collector was used to collect microdialysates and then was assayed by HPLC system.

Figure 2 showed the section of hippocampus, and arrow indicate the probe insert position.

**EXAMPLE 2: *In vitro* Microdialysis and *In vitro* green fluorescent protein gene (GFP) imaging system together with monitoring the differentiation process**

A microdialysis system consists of a basic microdialysis pump (CMA/100, CMA, Stockholm, Sweden), a fraction collector (CMA/140, CMA), and a microdialysis probe cemented to a Petri dish (Figure 3). The dialysis probe was made of a dialysis membrane 30 mm in length, 150 μm in outer diameter, and a nominal molecular weight cutoff of 13,000. Ringer solution (8.6 g/L NaCl, 0.3g/L KCl, and 0.33 g/L CaCl2) was perfused through the probe at a flow rate of 2μl/min. Microdialysate samples were collected every 10min. Each microdialysate of 10 μl was injected manually into the chromatographic system and was assayed on the same sampling day. The Liquid chromatography and electrochemical detection contains a model HTEC-500 LC-ED system (Eicom, Kyoto, Japan), consisting of a three-electrode cell with Ag/AgCl as the reference electrode, a counter- electrode, and a graphite electrode as the working electrode, were used to measure dopamine (DA) and serotonin (5-HT). The experiment employed a graphite working electrode with a 25-μm gasket with the applied potential set at +450mV versus Ag/AgCl. The column used for the separation of DA and 5-HT was a PP-ODS column (4.6×30 mm, Eicom) at room temperature (25 ˚C). The mobile phase, containing 0.1M sodium phosphate buffer (0.1M NaH2PO4/ 0.1M Na2HPO4, 1000:160, v/v), 1% methanol, 500mg/L sodium secane-sulfonate, and 50mg/L EDTA, was adjusted to pH 6.0 with 5M NaOH with a flow rate of 0.5ml/min.

As shown in Figure 3, the microdialysis system for Petri dish including guide cannulae (A); dialysis membrane (30 mm in length) (B); culture medium (C); petri dish cover (D); petri dish body (E); PE-10 (F); running foot tubing (G) and silica tubing (H). (3) The real-time evaluation system - simultaneously monitoring the differentiation process (the number of neurite, neurite length, and primary dendrites) of neural stem cells under the observations of CCD confocal microscope and the dopamine (DA) and serotonin (5-HT) concentrations by microdialysis with HPLC-ED.

As shown in Figure 4, the microdialysis device could be connected to automatic pump and automatic sample-collector under $CO_2$ cell incubator. This system, including $CO_2$ incubator, can provide continuously, long-term (one to weeks), real-time monitor cells or stem cells the process of proliferation and differentiation.

**EXAMPLE 3: monitoring Differentiation process of Neural Stem Cell by *in vitro* GFP imaging system.**

Figures 5 and 6. Neural stem cells (NSCs) isolated from the hippocampal region of SD rats were cultured in bFGF and EGF DMEM-serum free medium. After 1 week, NSCs formed the floating spheroid bodies, called "neurospheres". Then these neural stem cells/ neurospheres were stably expressed green fluorescent protein (GFP) after 3 days incubation with the viral supernatant of murine stem cell vector (MSCV) carried GFP (MSCV-GFP). We further detected the multi-potent differentiation ability of NSCs monitored by *in vitro* GFP signals using the device as described in Figure 4. As shown in Figure 5(A) Under 2% FBS DMEM medium for 24 hours culture, neuropheres still constitutively expressed GFP fluorescence and were attached to dish pre-coated with poly-L-ornithin/lysine. (B) After 72 hours, the neurospheroid-like NSCs differentiated into neuronal-like cells. As shown in Figure 6 (A), the differentiating (B) neuronal-like cells still expressed GFP fluorescence by using the device as described in Figure 4. Using immunofluorescent assay, the signals of GFP protein and thyrosine hydroxylase (TH) protein were detected in the same neuronal cell derived form NSC.

**Example 4: *in vivo* GFP Imaging**

The preparation of murine stem cell retroviral vectors- MIGFP, PMD, and VZV-G were stated in Shih Hwa Chiou et al., Childs Nerv Syst (2006) 22: 475-480. To generate retroviral supernatants, 293 cells were transiently transfected by calcium phosphate-mediated coprecipitation with 5 ug of the plasmids of MIGFP, PMD, and VZV-G. The cells were fed at 24 hours postinfection, and the retroviral supernatant was used at 48 hours. The cell continuous to produce high-titer retrovirus for another 2 days, and that supernatant was used if needed for additional experiments. The supernatant was collected, brought to 8 ug of polybrene per ml 10 mM HEPES, and filtered 0.45 um pore-sized filter for use. Stem cells for infection were washed and trypsinized. They then plated at 106 cells per well of a six-well dish and centrifuged. The stem cell medium was removed, and retroviral supernatant was added at 1 ml/$10^6$ cells.

**HPLC-ED assay**

Dopamine levels of cell lysate were determined by cells at day 14 of the fifth stage. The HPLC-ED system consisted of a chromatographic pump (BAS, PM-80, Bioanalytical System, West Lafayette, IN, USA) at a flow-rate of 0.06 ml/min for buspirone analysis using a cyano micro-bore column (BAS, Sepstik CN-51~, 150x 1 mm I.D., particle size 5 txm) in series after an on-line injector. The mobile phase consisted of 0.1 M monosodium dihydrogen orthophosphate-acetonitrile-diethyl-amine (85:15:0.1, v/v/v, pH 3.0 adjusted with orthophosphoric acid). The mixture was filtered with a 0.22-txm millipore membrane and degassed by helium. The injection volume was configured with a 10-p~1 sample loop on an on-line injector (CMA-160, CMA/Microdialysis, Stockholm, Sweden). Buspir- one was measured using an electrochemical detector (BAS 4C). The potential for the glassy carbon working electrode was set at + 1.10 V with respect to an Ag/AgC1 reference electrode. The output from the electrochemical detector was recorded using Waters Millennium 2020 software (Tsai-PC12-3).

**Transplantation and Behavior Testing**

6-OHDA lesion. A single dose of 6-OHDA was administrated intrabrain injection as described to 8-week-old nude mice (dose). All surgical procedures were done according to guidelines of Yang-Ming University and Taipei Veterans General Hospital. DHA-treated HSC were trypsinized at day 14 of induction, and re-suspended at a density of $10^5$ viable cells per ul. Ten microliters of the cell suspension were grafted into the lesioned striatum of hemiparkinsonian mice. In sham animals, 10 ul of N2 medium was injected. Amphetamine-induced (2.5 mg Kg) rotational behavior was evaluated for 70 min.

**Magnetic resonance imaging**

Mice were imaging during the 4-week period of stem cell growth at 3 Teslas (T) using a 21-cm horizontal-bore MR unit (Bruker, Billerica, MA) with 39 G/cm gradients and a 35-mm transmit-receive birdcage volume coil. MRI was performed using 2-dimensional (2D) T2-star-weight (T2*)-weighted gradient echo (TR/TE=400-450/3.8-4.2 ms, number of excitations)

The *in vivo* function of neural stem cells (NSCs)-derived neuronal subtypes were assessed in the nude mice with 6-hydroxy-dopamine (6-OHDA) induced lesions. After injected with 14 Days NSC for 6 weeks, the cell proliferation was detected by MRI (Figs. 7A and B), *in vivo* and *ex vivo* GFP tracing system. Because skin pigments and hair can significantly scatter light from underlying structures, hairless nude mice were used so the deep structures could be imaged. *In vivo* GFP (Fig. 7C-F) imaging enabled the continuous, real-time monitoring of signals and *ex vivo* GFP (Fig. 7F-H) imaging further confirmed the location of the injected and differentiated cells. The use of two imaging methods, MRI and GFP imaging, one had assessed delivery and the other had assessed efficacy. Together with the consistent imaging results, the two imaging approaches fully demonstrated the strengths of our experimental models. By dual immunohistostaining, we further found that most GFP expression was co-localized within the thyrosine hydroxylase-positive cells (Figure 7 I-K). Furthermore, using HPLC-ED assay (please see Fig. 8), we can functionally detect the neurotransmitters (5-HT and dopamine) released from the transplanted NSCs in the brain of nude mice. The illustration and study concept of whole monitoring system (GFP imaging, auto-sampling, and HPLC-ED systems) was shown in Figure 7L.

The incorporation of GFP reporter gene into the genome of NSCs was demonstrated as markers of transcription that response to dopaminergic differentiation. In addition, the example also showed that *in vivo* GFP imaging is a noninvasive means of tracking NSC in Parkinson's disease-like animal model. *In vivo* and Ex *vivo* GFP imaging are ideal imaging modality for understanding the parameters of gene delivery and the effective expression in model systems. Further, by using immunodeficient mice model, a model of physiologically relevant system to human could be successfully established and be used to investigate the function of stem cells. Combining with GFP imaging/auto-sampling/HPLC-ED systems, the proliferation process of stem cells and function-releasing neurotransmitters can be continuously monitored. Given the above, the example provided a real-time report gene-labeling system to monitor the treatment of stem cells for Parkinson's disease.

Figures 7A and 7B showed MRI scans acquired in axial (A) coronal (B) planes. The images were from a live mouse and signal from the skull and muscles were removed from the images. MRI images of mice brain images was obtained in 2T at approximately-$mm^3$ resolution. Figures 7C, 7D and 7E illustrated microscopic images: (C) Dissecting microscope (D) In vivo GFP imaging and (E) Ex vivo GFP imaging. Top views of the hairless nude mice, the noninvasive in vivo GFP imaging permitted real-time tracking of the delivered cells and the ex vivo GFP imaging provided more accurate information of the status and the location of the transplanted cells. Figures 7F, 7G and 7H illustrated digital microscopic images: (F) Microscopic (G) green fluorescence (H) F and G merged. Two images were merged to give more clear view of the experimental mice model brain tissue. Figure 7 (I-K) By dual immunohistostaining, we further found that protein expression of GFP (purple; nucleus) was co-localized within the thyrosine hydroxylase (brownish; cytoplasma)-positive cells. The illustration and test results of whole monitoring system (GFP imaging, auto-sampling, and HPLC-ED systems) were shown in Figure 7L.

**EXAMPLE 5: Chromatography**

Figure 8A is a chromatogram of standard solution of dopamine (DA) and serotonin (5-HT) each at a concentration of 100 ng/ml. Figure 8B is the chromatogram of dialysate collected from non-induced neural stem cell medium with a basal concentration of 6.04 and 0.18 ng/ml for DA and 5-HT, respectively. Figure 8C is the chromatogram of a dialysate sample containing DA 46.9 ng/ml and 5-HT 2.84 ng/ml collected after the induction with polyornithine/ laminin-coating plate and NGF of neural stem cell. The analytes were well separated using the present chromatographic conditions. It can be seen that the DA and 5-HT were well resolved and free of interference from dialysate in the culture medium, with retention times of 1.7 and 3.9 min, respectively. The total run time for the dialysate was 5 min. Compared with our previous report (Cheng et al., 2000), this result shortened the chromatographic run time from 30 min to 5 min and the metabolites of DA and 5-HT may not be retained on the column due to the unique design of the column material (PP-ODS, Eicom, Japan).

**Accuracy and precision**

The precision and accuracy of the assays were examined using a standard mixture of DA and 5-HT. The intra- and inter-assay variabilities were assessed with six replicates and expressed as the relative standard deviation (RSD %) for precision and bias % for accuracy. Inter- and intra-assay accuracy and precision of the analysis were less than 10 % in the concentration of 0.1, 5, and 100 ng/ml of DA and 5-HT (Tables 1 and 2).

Table 1. Intra- and inter- assay accuracy and precision for the determination of dopamine.

| Nominal Concentration (ng/ml) | Observed concentration (ng/ml) | RSD (%) | Bias (%) |
|---|---|---|---|
| Intra-assay | | | |
| 0.1 | 0.098±0.003 | 3.14 | -2.47 |
| 5.0 | 5.23±0.16 | 3.06 | 4.55 |
| 100 | 98.19±1.14 | 1.16 | -1.8 |
| Inter-assay | | | |
| 0.1 | 0.102±0.002 | 1.90 | 1.89 |
| 5.0 | 4.91±0.09 | 1.77 | -1.80 |

(continued)

| | | | |
|---|---|---|---|
| Inter-assay | | | |
| 100 | 98.21±1.02 | 1.04 | -1.79 |
| Observed concentrations are expressed as mean±S.D. | | | |

Table 2. Intra- and inter- assay accuracy and precision for the determination of 5-HT.

| Nominal Concentration (ng/ml) | Observed concentration (ng/ml) | RSD (%) | Bias (%) |
|---|---|---|---|
| Intra-assay | | | |
| 0.1 | 0.102±0.004 | 3.81 | 2.20 |
| 5.0 | 5.08±0.28 | 5.47 | 1.62 |
| 100 | 99.37±0.43 | 0.43 | -0.63 |
| Inter-assay | | | |
| 0.1 | 0.102±0.003 | 3.02 | 1.67 |
| 5.0 | 5.11±0.86 | 1.68 | 2.23 |
| 100 | 98.99±1.20 | 1.21 | -1.02 |
| Observed concentrations are expressed as mean±S.D. | | | |

**Linearity, recovery, limits of detection and quantitation**

Linear least-square regression analysis of the calibration graph demonstrated linearity between the response and the nominal concentration of DA and 5-HT over the range of 0.05-100 ng/ml. The linear equations are y=24504x+1603.2 and y=30537x+2566.6 for DA and 5-HT, respectively. The correlation coefficients were higher than 0.995. The vertical y axis is the peak area and the horizontal x axis is the amount of analytic concentration in ng/ml. The in vitro recoveries of DA and 5-HT in PC-12 culture medium of microdialysis probes were 31.62 ± 1.21% and 23.77 ± 1.05% for DA and 5-HT, respectively (mean±S.E.M.) based on the concentration of 25 and 50 ng/ml of DA and 5-HT. The limit of detection (LOD) was 0.01 ng/ml at signal-to-noise ratio of 3 for DA and 5-HT. The limit of quantitation (LOQ) was defined as the lowest concentration of linear range for DA and 5-HT and was 0.05 ng/ml.

**Example 6: Fluoxetine up-regulates expression of cellular FLICE-inhibitory protein and inhibits LPS-induced apoptosis in hippocampus-derived neural stem cell**

**MATERIALS AND METHODS**

**The isolation and culture of neural stem cell culture**

All animal used were treated in accordance with the Animal Care and Use Committee guideline at Taipei Veterans General Hospital. Adult Sprague-Dawley rats (8 weeks old, 250 g) were anesthetized with intraperitoneal phenobarbital (Sigma, USA). The location of their brains was mapped: then, the brain was surgically separated from the hippocampus region with the procedure. Tissues from the hippocampus of the adult SD rats were dissociated and incubated in Hank's balanced salt solution (HB'SS) containing collagenase (78 units/ml) and hyaluronidase (38 units/ml) for 10 mins at 37°C. The tissues were then mechanically dissected and placed in a trypsin solution (1.33 mg/ml) at 37°C for another 10 mins. Dissociated cells were then centrifuged at 150g for 5 mins. The enzyme solution was then removed and replaced with serum-free culture media composed of Dulbecco modified Eagle medium (DMEM) and F-12 nutrient (1:1) including insulin (25 μg/mL), transferrin (100 μg/mL), progesterone (20 nM), putrescine (60μM), sodium selenite (30 nM), and human recoFLinant epidermal growth factor (EGF) 20 ng/mL and fibroblast growth factor-basic (bFGF) 20 ng/mL (R&D Systems, Minneapolis, MN). Viable cells were counted by trypan blue exclusion and plated as 5000 cells/200 μL per well in 96-well plates (Corning, Acton, MA) with no substrate pretreatment.

**Cell viability assay and Enzyme-linked immunosorbent assay (ELISA)**

Fluoxetine was purchased from Sigma, Inc. (USA). NSC cells were seeded on 24-well plates at a density of $2\times10^4$ cells/well in medium, followed by methyl thiazol tetrazolium assay (MTT assay; Sigma-Aldrich Co.) for cell viability. NSC cells were incubated with 0.25 mg/ml MTT for 4 hours at 37°C and the reaction was terminated by the addition of 100% isopropanol. The amount of MTT fromazon product was determined by using a microplate reader and the absorbance was measured at 560 nm (SpectraMax 250, Molecular Devices, Sunnyvale, CA, USA). The activities of caspases 8 and 3 (Medical & Biological Laboratories Co., Ltd., Nagoya, Japan), and the concentration of IL-6, IL-1 and TNF-α (Pharmingen, USA) were determined by ELISA kits and quantified by reading at A490 nm (MRX; Dynatech Laboratories, Chantilly, VA). Each individual sample was analyzed in triplicate.

**Real-time reverse transcription-polymerase chain reaction (RT-PCR)**

For real-time RT-PCR, the total RNA was extracted using the $RNA_{easy}$ kit (Qiagen, Valencia, CA, USA) as previously described C.L. Kao et al., Mod Pathol 18 (2005) 769-778.[15]. Briefly, total RNA (1 μg) of each sample was reversely transcribed in 20μL using 0.5 μg of oligo dT and 200 U Superscript II RT (Invitrogen, Carlsbad, CA, USA). The amplification was carried out in a total volume of 20 μl containing 0.5 μM of each primer, 4 mM $MgCl_2$, 2 μl LightCycler™ -FastStart DNA Master SYBR green I (Roche Molecular Systems, Alameda, CA, USA) and 2 μl of 1:10 diluted cDNA. The quantification in the unknown samples was performed by the LightCycler Relative Quantification Software version 3.3 (Roche Molecular Systems, Alameda, CA, USA). In each experiment, the GAPDH housekeeping gene was amplified as a reference standard. GAPDH primers were designed: GAPDH(f): GGGCCAAAAGGGTCATC ATC (nt 414-434, GenBank accession no. BC059110.1), GAPDH(r): ATGACCTTGCCCACA GCCTT (nt 713-733). Other target gene primers were as follows: Bcl-2(f): GGGATGACTTCTCTCGTCGCTAC (nt 527-550, GenBank accession no. NM_016993.1), Bcl-2(r): GTTGTCCACCAGGGGTGACAT (nt 721-742). Bcl-xL(f): CAGCTTCATAT AACCCCAGGGAC (nt 402-425,GenBank accession no. U72350.1), Bcl-xL(r): GCTCTAG GTGGTCATTCAGGTAGG (nt 586-610). c-$FLIP_L$ (f) GCTGAAGTCA TCCATCAGGT (nt 488-509, GenBank accession no. NM_003879), c-$FLIP_L$(r): CATACTG AGATGCAAGAATT (nt 713-731). Fas(f): AGAGC TGTGGCTACCGGTGAT (nt 301-322, GenBank accession no. NM_ 012908.1), Fas(r): AGA GGGATGGACCTTGAGCG (nt 524-544). TRAIL-R2(f): CACCAGGTGTG ATTCAGGTG and TRAIL-R2(r): CCCCACTGTGCTTTGTACCT-3') (nt 492-712, GenBank accession no. AF016849). Reactions were prepared in duplicate and heated to 95°C for 10 mins followed by 40 cycles of denaturation at 95°C for 10 seconds, annealing at 55°C for 5 seconds, and extension at 72 °C for 20 seconds. All PCR reactions were performed in duplicate. Standard curves (cycle threshold values versus template concentration) were prepared for each target gene and for the endogenous reference (GAPDH) in each sample. To confirm the specificity of the PCR reaction, PCR products were electrophoresed on a 1.2 % agrose gel.

**The Plasmid and promoter luciferase assay**

The pGL3-FLIP was obtained from based on T. Bartke et al., Oncogene 20 (2001) 571-580. The c-FLIP promoter deletion constructs pGL3-FLIP978, pGL3-FLIP696, pGL3-FLIP414 and pGL3-FLIP132 were generated by PCR amplification of corresponding DNA sequences from pGL3-FLIP using the following forward primers 5'-GGAAGATCTTAAAAATACAAA AAAAAAAA-3' (-696), 5'-GGAAGATCTGTGGCTCACGCCTGTAATCC-3' (-414), 5'-GG A AGATCTAGAAAGAAAAAAAAAACACT-3' (-132) with the Bg1II site underlined and the reverse primer 5'-CCCAAGCTTGGTACCGAGCTCGGATCCAC-3' with the HindIII site underlined and insertion of each resulting PCR product into pGEMT vectors (Promega) through TA cloning. The corresponding PCR products were then digested with BglII and HindIII, and cloned into pGL3-basic between these two sites. Transfections, luciferase assays and Westerns were all performed as described T. Bartke et al., Oncogene 20 (2001) 571-580. For reporter assays using the PI3-K inhibitor, indicated amounts of LY294002 were added to the culturing medium 8 hours before harvesting cells.

**c-FLIP RNA Interference**

Double-stranded, short interfering RNA (siRNA) of the c-FLIP were chemically synthesized by Amnion Inc. (Austin, TX, USA) and the sense and antisense sequences were designed as follows: Sense: GGAUCCUUCAAAUAACUUCtt; Antisense: GAAGUUAUUUGAAGGAU CCtt, starting from nucleotide 1066 of c-FLIP sequence (accession number NM_003879). The transfection of siRNA was performed using Lipofectamine 2000 (Invitrogen) according to the manufacture's instructions. In brief, cells were harvested with 0.25% trypsin, 1 mM EDTA in phosphate buffered saline (PBS) without $Ca^{2+}$ and $Mg^{2+}$, and plated in six-well plates at $10^5$ per $cm^2$. Then 3.5 μl siRNA (100 μM solution) was mixed with 125 μl serum-free culture medium for 5 min at room temperature. During this incubation period, 5 μl of Lipofectamine 2000 was diluted in 125 μl serum-free culture medium. These two mixtures were combined, mixed gently, and incubated for 20 minutes at room temperature for complex formation. This 250 μl of siRNA-Lipofectamine mixture was then added into the cells in a final volume of 2.5 ml per well. The transfected cells were cultured and fed daily with fresh medium until they were assayed.

### Immunofluorescence Staining

An avidin-biotin complex method was used for the immunohistochemical staining in the differentiated NSCs. Following washes with 3% hydrogen peroxide and sodium azide, antigenicities were retrieved using a microwave. Each slide was then treated with antibodies for MAP2 (Chemicon, Temecula, CA, USA), GFAP (Chemicon, Temecula, CA, USA), c-$FLIP_L$ (sc-8347, Santa Cruz, USA), and serotonin (5-HT; polycolonal, Chemicon, Temecula, CA, USA). Immunoreactive signals were detected with a mixture of biotinylated rabbit antimouse IgG and Fluoesave (Calbiochem, La Jolla, CA, USA) under immunofluorescent microscope (Olympus FV300).

## RESULTS

### Fluoxetine activates c-FLIP promoter (-414 to -133 bp) region and the protein expression of c-Flip through PI3K associated pathway.

To determine the promoter region of the c-FLIP gene responsive to FL-mediated activation, the serially deleted c-FLIP promoter mutants (bp -978 to +150, bp -696 to +150, -414 to +150, and -132 to +150 were constructed and analyzed (Fig 9A). The results showed that the c-FLIP promoter region from bp -414 to -133 was necessary for its activation by FL (Fig. 9B, compare lanes 3-8 to 8-10). This finding supported that FL up-regulated the mRNA expression of c-FLIP was associated with the activation of c-FLIP promoter from bp -414 to -133 (Fig. 9B). In this region, the transcriptional factor sites including CREB and SP1 were found (Fig. 9A). Moreover, we found that the protein levels of inactive caspases 8 and 3 (pro-form) were not decreased during different concentrations of fluoxetine treatment. Using dual-immunostaining assays following the FL-treated effects on differentiated NSCs, we further observed that the c-$FLIP_L$ signal (green) on day 5 FL-treated NSC was exclusively present in the cytoplasm of MAP2-positive neuron (red) (Fig. 9D). The data further confirmed that c-$FLIP_L$ expression induced by FL was specifically inhibited by the PI3-K inhibitor (20, 50, and 100 M LY294002; Fig. 9E). This result indicated that FL up-regulated the mRNA and protein expression of c-FLIP was associated with PI3-K-dependent pathway.

### Example 7: *in vitro* Microdialysis

### Cell line and method

INS-1 cells were isolated from rat insulinoma, grown in 60 mm petri dish in high glucose RPMI 1640 medium with 2 mM L-glutamine adjusted to contain 1.5 g/L sodium bicarbonate, 4.5 g/L glucose, 10 mM HEPES, and 1.0 mM sodium pyruvate, 90%; fetal bovine serum (FBS), 10%, and maintained in a 5% $CO_2$ humidified incubator at 37. When the petri dish was filled with INS-1, cell were transferred to polypropylene centrifuge tube with screw cap and cultured with high glucose RPMI medium.

The cell viability and proliferation of INS-1 were analyzed by using the MTT assay. Study devised cell into two group, the control group did not treated with any compound, the experiment group treated with candidate drugs. Sampling the 0 to 4 day medium by microdialysis collector and analyzed with HPLC system.

### Microdialysis system

As shown in Figure 10, the sampling device was perfused with the blank RPMI medium as perfusate at 2 ml per min by a micropump (CMA-400, CMA, Stockholm, Sweden). Microdialysates were introduced by an microfraction collector (CMA-142) and analyzed by HPLC at 12 min intervals. The equipped microfraction collector (CMA-142) was used to collect microdialysates. Fifty ml of aliquots of microdialysates were assayed for insulin by HPLC system while the polypropylene centrifuge tube was kept in an $CO_2$ incubator. The microdialysates was filtered through a 0.22-mm cellulose nitrate filter membrane (Sartorius, Germany), then directly injected (50 $\mu$L) onto the HPLC column.

As dialyzing efficiency increases with increase in surface area, a relatively long microdialysis probe was constructed. In brief, a 9.5 cm section of capillary silica tubing was inserted into a 1 cm running foot tubing. A 8 cm section of polyethylene PE-10 tube (0.28 mm i.d.; 0.61 mm o.d., Clay Adams, MD, USA) and a 5-cm piece of dialyzing membrane (Spectrum hoilow dialysis membrane, 150 pm diameter, nominal molecular weight cut-off 100000 Da) were concentrically inserted into the running foot tubing. All tubing unions and the end of the dialysis membrane were cemented with epoxy. At least 24 hrs were allowed for the epoxy to dry.

### HPLC condition

The chromatographic system consisted of an Chromolith® Performance RP-18 endcapped column (100 x 4.6 mm), a WATERS UV-Vis detector, an autosampler (Shimadzu, SIL-9A ). The software SmartChrom (v.2.12) was used to pilot the HPLC instrument and to process the data throughout the method validation and sample analysis. HPLC grade acetonitrile and trifluoroacetic acid (TFA) were obtained from Merck (Darmstadt, Germany). Insulin and nateglinide standard was purchased from sigma. Standard in medium were prepared by mixing the appropriate volumes of solutions of insulin, to obtain a standard medium containing insulin at a concentration of 0.1-20 μg /ml. Dilutions were prepared with a blank medium. Standard solution was kept separately at -20°C to prevent its possible decomposition in the standard calibration samples. The mobile phase consisted 30/70 (v/v) acetonitrile/water with 0.07 % trifluoroacetic acid (TFA) and adjusted pH=2.5 with $H_3PO_4$ buffer. An aliquot of 50 μL of the sample was injected onto the HPLC column. The flow-rate was 1.0 ml/min at room temperature. The chromatographic separation was simultaneously monitored at UV 215 and 270 nm.

**In vitro microdialysis recovery**

An in vitro experiment was performed to calculate the relative recovery and variability of the microdialysis probes. Prior to the experiments, in vitro recovery was performed using a standard mixture containing insulin, to determine the recoveries of all analytes of the microdialysis system. First a microdialysis probe was placed in culture media containing candidate drugs. The relative recovery (recovery $_{in\ vitro}$=$C_{out}$/$C_{medium}$, $C_{out}$ is the concentration of each analyte, and $C_{medium}$ is the concentration of the analyte in the polypropylene centrifuge tube) was calculated by comparing the area under the peaks in the UV detector chromatogram for insulin.

**Example 8: Quantitative determination of unbound cefoperazone in rat bile using microdialysis and liquid chromatography Chemicals and reagents**

Cefoperazone was purchased from Pfizer (Roma, Italy). Berberine injection (5 mg/ml) was purchased from Kyorin (Taoyuan, Taiwan). Liquid chromatographic grade solvents and reagents were obtained from E. Merck (Darmstadt, Germany). Triple de-ionized water (Millipore, Bedford, MA, USA) was used for all preparations.

Animals

Adult, male Sprague-Dawley rats (280-350 g) were obtained from the Laboratory Animal Center at National Yang-Ming University (Taipei, Taiwan). These animals were specifically pathogen-free and were allowed to acclimate to their environmentally controlled quarters (24 ± 1°C and 12:12 h light-dark cycle) for at least 5 days before the experiments began. At the start of experiments, the rats were anesthetized with urethane 0.8 g/ml and chloralose 0.08 g/ml (0.1 ml/kg, i.p.). Throughout the experimental period, anesthesia was maintained by administering one quarter of the initial dose at each hour. The experimental animals were kept warm with a heating pad throughout the experiments.

Chromatography

The HPLC system consisted of a chromatographic pump (BAS PM-80, Bioanalytical System, West Lafayette, IN, USA), an on-line injector (CMA/160, Stockholm, Sweden) equipped with a 20 μl sample loop and a UV detector (Soma S-3702, Tokyo, Japan). Cefoperazone dialysate was separated using a LiChrosorb RP-18 column (Merck, 250 mm × 4.6 mm i.d.; particle size 5 μm) maintained at ambient temperature. The mobile phase comprised 100 mM monosodium phosphoric acid (pH 5.5)-methanol (70:30, v/v), and the flow rate of the mobile phase was 1 ml/min. The buffer was filtered through a Millipore 0.45 μm filter and degassed prior to use. Detecting UV wavelength was set at 254 nm. Output signal from the HPLC-UV was recorded using an EZChrom chromatographic data system (Scientific Software, San Ramon, CA, USA).

Method validation

All calibration curves of cefoperazone (external standards) were made prior to the experiments with correlation values of at least 0.995. The intra-day and inter-day variabilities for cefoperazone were assayed (six replicates) at concentrations of 0.5, 2, 10, 50, 250, 1000 and 4000 μg/ml on the same day and on six consecutive days, respectively. The accuracy (% Bias) was calculated from the nominal concentration ($C_{nom}$) and the mean value of observed concentration ($C_{obs}$) as follows: bias (%)= [($C_{nom}$ - $C_{obs}$)/($C_{nom}$)]× 100. The relative standard deviation (R.S.D.) was calculated from the observed concentrations as follows: % R.S.D. = [standard deviation (S.D.)/$C_{obs}$]× 100. Accuracy (% Bias) and precision (% R.S.D.) values of within 15% cowering the range of actual experimental concentrations were considered acceptable.

Microdialysis experiment

The bile duct microdialysis probes were manually made in-house based on the design originally described by Scott and Lunte (Scott D.O., Lunte C.E., Pharm. Res., Volume: 10, (1993), pp. 335-342(. The detailed construction of the flow-through microdialysis probe has been described. In brief, a 7-cm piece of dialysis membrane (spectrum, 150 μm outer diameter with a cut-off at nominal molecular weight of 9000, Laguna Hills, CA, USA) was inserted into a section of the polyethylene tubing (PE-60; 0.76 mm i.d.; 1.22 mm o.d.), with the ends of the dialysis membrane connected to a piece of silica tubing (40 μm i.d; 140 μm o.d., SGE, Australia). A piece of PE-10 tubing (0.28 mm i.d.; 0.61 mm o.d.) was then attached to both ends of the PE-60 tubing and all unions were cemented with epoxy. At least 24 h was allowed for the epoxy to dry. After bile duct cannulation, the probe was perfused with normal saline and the flow rate set at 2 μl/min. Outflows from the bile microdialysis probe were connected to an on-line injector and automatically injected every 10 min. After dialysate levels had stabilized (approximately 2 h), cefoperazone (30 mg/kg) was intravenously administered via the femoral vein. From each sample, 20 μl of dialysate was assayed using the HPLC system. In the pretreatment group, 30 mg/kg berberine was given intravenous 24 h before cefoperazone administration.

Recovery of microdialysate

For in vivo recovery, normal saline solution containing cefoperazone (50 or 100 μg/ml) were pumped through the probes at a constant flow rate (2 μl/min) using the infusion pump (CMA/100). After a stabilization period of 2 h, the inlet ($C_{in}$) and outlet ($C_{out}$) concentrations of cefoperazone were determined by HPLC. The in vivo recovery ratios were then calculated by the following equation:

$$\text{Recovery}_{\text{in vivo}} = 1 - (C_{\text{out}} / C_{\text{in}}).$$

Drug administration

After a 2-h post-surgical stabilization period, cefoperazone (30 mg/kg) was administered via femoral vein by i.v. bolus injection for each rat. Berberine (30 mg/kg) was pretreated 24 h before cefoperazone administration in the berberine group. Six animals were used in each group. All dialysates were collected every 10 min and then measured by a validated HPLC system.

Pharmacokinetic study

The concentrations of cefoperazone in the rat bile dialysates were determined from the calibration curves. Absolute concentrations in extracellular fluid were calculated from the concentrations in dialysates by the following equation: concentration = dialysate/recovery. Pharmacokinetic calculations were performed using the observed data. All data were subsequently processed by the computer pharmacokinetic program WinNonlin standard version 1.1 (Science Consulting Inc., Apex, NC, USA) for the calculation of pharmacokinetic parameters according to the non-compartmental model. All data are presented as mean ± standard error. The area under the concentration curves (AUC), the area under the first moment curve (AUMC) and the mean residence time (MRT) were calculated by using statistical moments. The mean residence time and clearance was calculated as follows: MRT = AUMC/AUC, CL = Dose/AUC. The $t$ -test was employed and the level of significance was set to $p < 0.05$.

Results and discussion

The chromatograms obtained evolving from the liquid chromatographic method shown in Fig. 13. Each analysis of the microdialysate was completed within 10 min. Separation of cefoperazone from endogenous chemicals in dialysates from biliary duct was achieved in an optimal mobile phase containing 70% of 100 mM monosodium phosphate (pH 5.5) and 30% of methanol. The retention time of cefoperazone was 6.0 min (Fig. 13). Peak areas of cefoperazone were linear ($r^2 > 0.995$) over a concentration range from 0.5 to 4000 μg/ml, Fig. 13A shows a typical chromatogram of a standard mixture containing cefoperazone (50 μg/ml). The blank sample (Fig. 13B) shows that the chromatographic conditions revealed no biological substances that would interfere significantly with the determination of cefoperazone. Fig. 13C depicts a chromatogram of microdialysate from rat biliary duct. The sample contains cefoperazone (27.8 μg/ml) collected from the microdialysates at 360 min following cefoperazone intravenous administration (30 mg/kg).

**Example 9: Neuroprotection by Imipramine Against Lipopolysaccharide-induced Apoptosis in Hippocampus-derived Neural Stem Cells Mediated by Activation of BDNF and the MAPK pathway**

**Materials and Methods**

**1. The isolation and culture of NSCs**

All animals used were treated in accordance with the Animal Care and Use Committee guideline at Taipei Veterans General Hospital. The protocol of the present study was also approved by the committee. Adult Sprague-Dawley (SD) rats (8 weeks old, 250 g) were anesthetized with intraperitoneal phenobarbital (Sigma, USA), and the location of their brains was mapped. Then, the brain was surgically separated from the hippocampus region. In brief, tissues from the hippocampus of adult rats were dissociated and incubated in Hank's balanced salt solution (HBSS) containing collagenase (78 units/ml) and hyaluronidase (38 units/ml) for 10 minutes at 37˚C. The tissues were then mechanically dissected and placed in a trypsin solution (1.33 mg/ml) at 37˚C for another 10 minutes. Dissociated cells were then centrifuged at 150 GG for 5 minutes. Then the enzyme solution was removed and replaced with serum-free culture media composed of Dulbecco Modified Eagle Medium (DMEM) and F-12 nutrient mixture (1:1) including insulin (25 μg/mL), transferrin (100 μg/mL), progesterone (20 nM), putrescine (60 μM), sodium selenite (30 nM), human recombinant epidermal growth factor (EGF) 20 ng/mL, and fibroblast growth factor-basic (bFGF) (20 ng/mL) (R&D Systems, Minneapolis, MN, USA). Viable cells were counted by trypan blue exclusion and plated as 5000 cells/200 μL per well in 96-well plates (Coming Inc, Acton, MA, USA) with no substrate pretreatment. The IM was purchased from Sigma Chem. Co., St Louis, MO, USA. The 0.1% dimethyl sulfoxide (DMSO) (Sigma Chem. Co., St Louis, MO, USA) was used as a vehicle control in all procedures and studies. For differentiation protocol, the neurosphere NSCs were plated on poly-L-ornithine-coated (15 μg/mL) glass coverslips in individual wells of 24-well plates (1.0 mL/well) in DMEM/F-12 medium containing 2% fetal calf serum (FCS) (Gibcol-BRL, Gaitherburg, MD, USA) and withdrawing EGF and bFGF. At the time of NSCs seeding, vehicle control or IM treatment was concurrently added.

**2. Cell viability assay and Enzyme-linked immunosorbent assay (ELISA)**

IM was purchased from Sigma Chem. Co., St Louis, MO, USA. NSCs were seeded on 24-well plates at a density of $2\times10^4$ cells/well in medium, followed by methyl thiazol tetrazolium assay (MTT assay) (Sigma-Aldrich Co., St Louis, MO, USA) for cell viability. NSCs were incubated with 0.25 mg/ml MTT for 4 hours at 37 ˚C and the reaction was terminated by the addition of 100% isopropanol. The amount of MTT fromazon product was determined using a microplate reader and the absorbance was measured at 560 nm (SpectraMax 250, Molecular Devices, Sunnyvale, CA, USA). NSCs were exposed to 0, 1, 2, 5, 10, 20, 30, 40, 50, 100, 200, and 300 μM of IM for 1, 3, 5, and 7 days, respectively. The activities of caspases 8 and 3 (Medical and Biological Laboratories Co., Ltd., Nagoya, Japan), and the concentration of IL-6, IL-1 and TNF-α (Pharmingen, San Diego, CA, USA) were determined by ELISA kits and quantified by reading at 490 nm (MRX; Dynatech Laboratories, Chantilly, VA, USA). Each individual sample was analyzed in triplicate.

**3. Real-time reverse transcription-polymerase chain reaction (RT-PCR)**

For real-time RT-PCR, the total RNA was extracted using the $RNA_{easy}$ kit (Qiagen, Valencia, CA, USA). Briefly, total RNA (1 μg) of each sample was reverse transcribed in 20 μL using 0.5 μg of oligo dT and 200 U Superscript II RT (Invitrogen, Carlsbad, CA, USA). The amplification was carried out in a total volume of 20 μl containing 0.5 μM of each primer, 4 mM $MgCl_2$, 2 μl LightCycler™ FastStart DNA Master SYBR green I (Roche Molecular Systems, Alameda, CA, USA) and 2 μl of 1:10 diluted cDNA. The quantification in the unknown samples was performed with LightCycler Relative Quantification Software version 3.3 (Roche Molecular Systems, Alameda, CA, USA). In each experiment, the GAPDH housekeeping gene was amplified as a reference standard. GAPDH primers were designed: GAPDH(f): GGGCCAAAAGGGTCATCATC (nt 414-434, GenBank accession no. BC059110.1), GAPDH(r): ATGACCTTGCCCACA GCCTT (nt 713-733). Other target gene primers were as follows: BDNF(f) 5'-CCAAGGCAGGTTCAA GAG-3'. BDNF(r): 5'-CCAGCAGAAAGAGAAGAGG-3' (nt 146-331, GenBank accession no.NM_170735). Bcl-2(f): GGGATGACTTCTCTCGTCGCTAC (nt 527-550, GenBank accession no. NM_016993.1), Bcl-2(r): GTTGTCCA CCAGGGGTGACAT (nt 721-742). Bax(f): GTGGT TGCCCTCTTCTACTTTGC (nt 328-351, GenBank accession no. NM_017059.1), Bax(r): GAGGACT CCAGCCACAAAGATG (nt 522-544). MAP2(f): GTTTACATTGTTCAGGAC CTCATGG (nt 316-341); MAP2 (r): TCGGTAAGAAAGCC AGTGTGGT (nt 551-573. GenBank accession no. NM_U12008.1). Reactions were prepared in duplicate and heated to 95˚C for 10 minutes followed by 40 cycles of denaturation at 95˚C for 10 seconds, annealing at 55˚C for 5 seconds, and extension at 72˚C for 20 seconds. All PCR reactions were performed in duplicate. Standard curves (cycle threshold values versus template concentration) were prepared for each target gene and for the endogenous reference (GAPDH) in each sample. To confirm the specificity of the PCR reaction, PCR products were electrophoresed on a 1.2 % agarose gel.

**4. Immunoblot analysis**

After the treatment with IM, the cell lysates of the NSCs were collected and the concentration of protein was determined by using the Protein Assay kit (Bio-Rad, Hercules, CA, USA). Cell extracts with sample buffer were boiled for 5 minutes and then separated by 10 % SDS-PAGE gel. After electrophoresis, the gel was transferred onto a PVDF membrane for immunoblotting. The membrane was first blocked by incubation in non-fat milk at room temperature for 2 hours, then incubated with anti-Bcl-2 antibody (Upstate Biotechnology Inc, Waltham, MA, USA), and anti-β actin (Chemicon International Inc, Temecula, CA, USA) for 2 hours at room temperature, washed fives times with Tris-Buffered Saline Tween-20 (TBST), and then incubated further at room temperature with horseradish peroxidase-conjugated secondary antibody for 2 hours. Then the membrane was washed six times with TBST and specific bands were made visible by chemiluminescence (Santa Cruz Biotechnology, Santa Cruz, CA, USA).

**5. BDNF and Bcl-2 RNA Interference**

Double-stranded, short interfering RNA (siRNA) of BDNF and Bcl-2 were chemically synthesized by Ambion Inc., Austin, TX, USA). The sense and antisense sequences of BDNF were designed as follows: Sense: GATCCCCGAGCTGTTGGATGAGGACCtt; Antisense: AGCTTAAAAAGAG CTGtt, starting from nucleotide 137 of the Bcl-2 sequence (accession number L14680). The sense and antisense sequences of Bcl-2 were designed as follows: Sense: GCGCUGGAUAUAACUUCUUtt; Antisense: AAGAAGUUAUAUC CAGCGCtt, starting from nucleotide 137 of the Bcl-2 sequence (accession number L14680). In this test, the siRNA control used was siRNA-Cy3 (QIAGEN; sense sequence: UUCUCCGAACGUGU CACGUdTdT-3'-Cy3 and the complement 5'-ACGUGACACGUUCGGAGAAdTdT-3'-Cy3) as an irrelevant control. The transfection of siRNA was performed using Lipofectamine 2000 (Invitrogen, Carlsbad, CA, USA) according to the manufacturer's instructions. In brief, cells were harvested with 0.25% trypsin, 1 mM ethylenediaminetetraacetic acid (EDTA) in phosphate buffered saline (PBS) without $Ca^{2+}$ and $Mg^{2+}$, and plated in six-well plates at $10^5$ per $cm^2$. Then 3.5 μl siRNA (100 μM solution) was mixed with 125 μl serum-free culture medium for 5 minutes at room temperature. During this incubation period, 5 μl of Lipofectamine 2000 was diluted in 125 μl serum-free culture medium. These two mixtures were combined, mixed gently, and incubated for 20 minutes at room temperature for complex formation. This 250 μl of siRNA-Lipofectamine mixture was then added into the cells in a final volume of 2.5 ml per well. The transfected cells were cultured and fed daily with fresh medium until they were assayed.

**6. Immunofluorescent Staining and terminal dUTP nick-end labeling (TUNEL) assay**

An avidin-biotin complex method was used for the immunohistochemical staining in the differentiated NSCs. Following washes with the 3% hydrogen peroxide, sodium azide and antigens were retrieved using a microwave. Each slide was then treated with antibodies for Bc1-2 (Upstate Biotechnology, Waltham, MA, USA), Nestin (Chemicon, Temecula, CA, USA), 4', 6-diamidino-2-phenylindole (DAPI; Chemicon, Temecula, CA, USA) and MAP2 (Chemicon, Temecula, CA, USA). Immunoreactive signals were detected with a mixture of biotinylated rabbit anti-mouse IgG and Fluoresave (Calbiochem, La Jolla, CA, USA) under confocal microscopy (Olympus, FV300). Each individual sample was analyzed in triplicate. Furthermore, apoptotic cells were identified by the TUNEL method (*In Situ* Cell Death Detection Kit, POD, Roche Boehringer Mannheim Corp., IN, USA) as described in Chiou et al., J. Immunol. 167, 4098-4103, 2001. Briefly, the cells with cover slips were washed with 1X phosphate buffered saline (PBS), fixed with 4% of paraformaldehyde for 10 minutes, permeabilized with 0.1 % of triton X-100 for 5 mins, and incubated with the TUNEL reagent provided for 1 hour. Chromogenic development was then applied with 3-amino-9-ethyl-carbazole, and slides were counterstained with H & E stain. The percentage of TUNEL-positive cells was measured by the average of 6 different areas as compared to the total cell number in the same section slide (under a x200 power field of a light microscope).

**7. Microdialysis and HPLC-ECD**

A microdialysis sampling system connected to the Petri dish was constructed in our laboratory according to the design originally described by Cheng et al., J. Chromatogr. A. 870, 405-411, 2000. The dialysis probe was made of a dialysis membrane 30 mm in length, with an outer diameter of 150 μm, and a nominal molecular weight cut-off of 13000. Ringer solution (8.6 g/l NaCl, 0.3 g/l KCl and 0.33 g/l $CaCl_2$) was perfused through the probe at a flow-rate of 2 μl/min. Microdialysate samples were collected every 10 minutes. An HPLC-ED system (HTEC-500; Eicom, Kyoto, Japan) consisting of a three-electrode cell with an Ag/AgCl electrode as the reference electrode, a counter electrode and a graphite electrode as the working electrode was used to measure determine the levels of serotonin (5-HT). The graphite working electrode had a 25 μm gasket and the applied potential was set at +450 mV vs Ag/AgCl. The column used for separation at room temperature (25 °C) was a PP-ODS (4.6 x 30 mm) (Eicom, Kyoto, Japan). The mobile phase, containing 0.1 M sodium phosphate buffer (0.1 M $NaH_2PO_4$ : 0.1 M $Na_2HPO_4$ = 1000:160, v/v), 1% methanol, 500 mg/l sodium secanesulfonate and 50 mg/l EDTA, was adjusted to pH 6.0 with 5 M of NaOH with a flow rate of 0.5 ml/min. A volume of 10 μl of each microdialysate was injected manually into the chromatographic system and assayed on the same sampling day.

**8. Statistical analysis**

The results were reported as the mean ± S.E.M. Statistical analysis was performed using the one-way or two-way ANOVA test followed by Tukey's test, or by Student's t test, as appropriate. A p<0.05 was considered to be statistically significant.

Results

**1. IM modulates the cell viability of NSCs**

To investigate the role of IM in the cell survival rate on hippocampal progenitors *in vitro,* NSCs isolated from the hippocampus of adult rats were cultured in the DMEM/F-12 serum-free medium with EGF and bFGF. After being cultured for 1 week, NSCs aggregated and formed the spheroid-like bodies called neurospheres (Fig. 14A). By using the immune fluorescence assay, positive signals of nestin (red color, a neuroprogenitor marker; Fig. 14B), 4', 6-diamidino-2-phenylindole (DAPI) (cell nuclei; Fig. 14C) and merged images (Fig. 14D) were highly expressed and detected in neurosphere-like NSCs. To investigate the neuron-lineage differentiation, the neurospheres were further cultured in the induction medium with 2% fetal bovine serum (FBS) for 14 days. The signals of microtubular associated protein-2 (MAP-2; a neuron marker; Fig. 14F) were detected in differentiated NSCs (Fig 14E). Furthermore, the cell survival rate of NSCs was analyzed using the MTT assay. NSCs were exposed to 0, 1, 2, 3, 4, 5, 10, 20, 30, 40, 50, 100 and 200 μM of IM for 3, 5, and 7 days, respectively (Fig. 14G, H, and I). The data showed the cell survival was significantly increased in 3 μM IM-treated NSC cells at Day 3, Day 5 and Day 7 cultures (p<0.05; Fig. 14G, H, and I). Since IM at 3 μM was shown to increase cell survival, the concentration of 3 μM IM was chosen for all further experiments.

**2. Detection of Bcl-2 expression in IM-treated NSCs by real-time RT-PCR and western blot**

To further investigate whether anti-apoptosis (BDNF and Bcl-2), apoptosis (Bax and Bad), and neuron-lineage differentiation (MAP2) play critical roles in the differentiation process in IM-treated NSCs, mRNA expression of Bcl-2, Bax, Bad, and MAP-2 was detected in the Day 3, Day 5 and Day 7 samples. By using a real time RT-PCR method, the results showed that the mRNA levels of Bcl-2 (Fig. 15A) and MAP2 (Fig. 15D) in Days 5 and Day 7 IM-treated NSCs were significantly increased compared to non IM-treated NSCs (p<0.05). However, the expressions of Bad and Bax were not activated on Day 3, Day 5, and Day 7 of IM-treated NSCs (Fig. 15B and C). Furthermore, compared to Day 7 non-IM-treated NSCs, the levels of Bcl-2 mRNA were significantly increased in the 1, 3, and 5 μM Day 7 IM-treated NSC groups.

To evaluate the protein expression of Bcl-2 in IM-treated NSCs, the western blot method revealed the protein levels of Bc1-2 in Day 3 IM-treated NSCs were higher than those in Day 3 non-IM-treated NSCs (as a control; p<0.05; Fig. 16A). The expression levels of the Bcl-2 protein were significantly increased in Day 7 IM-treated NSCs as compared to Day 3 or Day 5 IM-treated NSCs (p<0.05; Fig. 16A). Moreover, immunohistochemistry confirmed that the protein expression of Bc1-2 in the Day 7 IM-treated NSCs (Fig. 16B) was significantly increased compared to the NSCs without the IM treatment (Fig. 16B). The protein expression of Bc1-2 in Day 7 IM-treated NSCs was further inhibited by transfection with Bc1-2 siRNA (Figs. 16A and B), but was not inhibited by siRNA control (Fig. 16B). To further determine the roles of BDNF and the MAPK/ERK signaling pathway between IM treatment and Bcl-2 in NSCs, BDNF siRNA and the MAPK inhibitor-U0126 were used in IM-treated NSCs. The result showed that treatment with either IM or BDNF recombinant protein can activate Bcl-2 expression in NSCs (Fig. 16C). Furthermore, the Bcl-2 expression induced by IM in NSCs was effectively blocked by BDNF siRNA or U0126 (Fig. 16C). Indeed, this result supported that BDNF and the MAPK/ERK signaling pathway are the upstream regulators in the modulation of Bcl-2 gene transcription and protein expression in IM-treated NSCs.

**3. IM treatment protects LPS-induced apoptosis in NSCs mediated by the regulation of BDNF, the MAPK/ERK pathway, and Bcl-2.**

LPS is a highly proinflammatory component of the outer member of gram-negative bacteria and has been used as a stress model for inducing caspase-dependent apoptosis (Franchi et al., Biochem. Biophys. Res. Commun. 314, 377-381, 2003; Yu et al., Faseb. J. 19, 1822-1835, 2005). To investigate the neuroprotective role of IM in NSCs, the cell viabilities and anti-apoptosis activities in LPS-treated NSCs with or without IM treatment were evaluated. Compared to the only LPS-treated NSCs, BDNF or IM treatment significantly increases cell survival rates in the LPS-treated NSCs (Figs. 17A and B). In contrast, the cell viability in the LPS-treated NSCs with IM was dramatically decreased after added treatment with BDNF siRNA, U0126, and Bcl-2 siRNA, respectively (Fig. 17B). Furthermore, the protein expression level of Bc1-2 in the 3 μM IM- LPS-treated NSC group was significantly increased compared with that in the groups of 1, 2 μM IM-LPS-treated NSCs and only LPS-treated NSC and the control groups (Fig. 17C). By using transfections of Bcl-2 siRNA and siRNA control, we further confirmed that IM treatment can increase the survival rate in LPS-treated NSCs through the regulation of Bcl-2 (Fig. 17C).

Bcl-2, an anti-apoptotic protein, has previously been identified as a neuronal cell death repressor. In Figs. 15 and 16, the data confirmed that IM treatment can activate the mRNA and protein expression of Bcl-2 in IM-treated NSCs. Thus, we further attempted to validate IM treatment possesses the neuroprotective function and effect against LPS-induced apoptosis in IM-treated NSCs. Under LPS (100 ng/ml) exposure, we found that the apoptotic activities of caspase 8 and 3 in the Day 7 IM-treated NSCs groups were significantly decreased compared with those in the NSCs without IM-treatment group ($p<0.05$; Day 7 in Fig. 18). Consistent with the caspase-8 and -3 data, the results of TUNEL assay further confirmed that the IM treatment protected against LPS-induced apoptosis in NSCs, but not in the NSCs without IM-treatment group (Fig. 18). Furthermore, to investigate whether the inhibition of BDNF, the MAPK/ERK pathway, and Bcl-2 increased the LPS-induced apoptosis in IM-treated NSCs, the gene silencing with siRNA method (BDNF siRNA and Bcl-2 siRNA) and the MAPK/ERK inhibitor-U0126 were used. Consistently with the results of caspases 8, caspase 3, and TUNEL assays, the individual treatment with BDNF siRNA or Bcl-2 siRNA, or the MAPK inhibitor-U0126 significantly enhanced the apoptotic activities in combination with the LPS- and IM-treated NSCs (Fig. 18A-C). In contrast to the inhibitory effects of BDNF and Bcl-2 siRNA, the apoptosis activities in the siRNA control-transfected groups were dramatically lower than those in the BDNF or Bcl-2 siRNA-transfected groups (Fig. 18A-C; $p<0.05$). These results confirmed that IM prevents the LPS-induced apoptosis in NSCs mediated by the regulation of BDNF, MAPK/ERK, and Bcl-2.

### 4. IM inhibits the LPS-induced proinflammatory effects in NSCs mediated by the regulation of BDNF, U0126, and Bc1-2.

To investigate the anti-inflammatory effects of 3 μM IM in the NSCs, the LPS-induced model was used and the levels of IL-1β, IL-6, and TNF-α were examined by ELISA. The results showed that the levels of IL-1β, IL-6, and TNF-α in the LPS-treated NSCs were significantly increased compared with the control group (NSCs only; $p<0.05$; Fig. 18A, B, and C). Moreover, 3 μM IM treatment effectively reduces IL-1β, IL-6, and TNF-α production in the LPS-treated NSCs ($p<0.05$; Fig. 19A, B, and C). To further evaluate the anti-inflammatory role of BDNF, MAPK/ERK, and Bcl-2 in IM-treated NSCs, the gene silencing with the siRNA method (BDNF siRNA and Bcl-2 siRNA) and the MAPK inhibitor-U0126 were used, The treatment with BDNF siRNA, Bcl-2 siRNA, or the MAPK inhibitor-U0126 significantly increased the levels of IL-1β, IL-6, and TNF-α in LPS-treated NSCs with IM (Figs. 19A-C). In contrast to the inhibition effect of BDNF and Bcl-2 siRNA, the levels of IL-6, IL-1 and TNF-α in the siRNA control-transfected groups were significantly lower than those in the Bcl-2 siRNA-transfected groups (Figs. 19A-C; $p<0.05$). These data support that BDNF, the MAPK/ERK pathway, and Bcl-2 playing roles as the major effectors to against LPS-induced apoptosis in IM-treated NSCs.

### 5. IM facilitates NSCs differentiated into serotoninergic neurons

To further determine whether IM can promote NSCs differentiation into serotoninergic neurons, the IM-treated NSCs were cultured in a polyornithine-coating plate with 2% FBS. By using triple-staining immunofluorescent assay, the signals of MAP-2 (a neuron marker; Figs 20A, C, G, and I) and serotonin (5-HT; Figs. 20D, F, G, and I), were detected and co-localized in the same differentiated NSCs (DAPI: nuclei staining; Figs. 20B, C, E, F, H, and I). As shown in Figs. 20J, K, and L, the percentages of MAP-2- and serotonin (5-HT)-positive cells in the Day 7 culture of IM-treated NSCs were significantly increased compared to the non IM-treated NSCs ($p<0.05$). We then attempted to determine whether BDNF, MAPK/ERK, and Bcl-2 play the roles in the regulation of neuroplasticity and differentiation in IM-treated NSCs. The results further provided evidence that the treatment with BDNF siRNA, Bcl-2 siRNA, or the MAPK inhibitor-U0126 could significantly decrease the percentages of both MAP-2- and serotonin (5-HT)-positive cells in the Day 7 culture of IM-treated NSCs (Fig. 20A-C). Together, the data suggest that IM can facilitate NSCs to differentiate into serotoninergic neurons, in part mediated by BDNF, the MAPK/ERK pathway, and Bc1-2 regulation.

### 6. Detection of 5-HT concentration released from the IM-treated NSCs by HPLC

To further identify the functional production of serotonin (5-HT) in these NSC-derived neurons, microdialysis coupled with HPLC-ECD was used to measure the concentration of 5-HT in the medium of the IM-treated NSCs (Fig. 21A). A sample collected from IM-treated NSC medium yielded: 3,4-dihydroxyphenylacetic acid (DOPAC) (5.23 min), DA (6.87 min), 5-hydroxyindoleacetic acid (5-HIAA) (7.78 min), 4-hydroxy-3-methoxyphenylacetic acid (HVA) (10.90 min) and 5-HT (15.67 min). The analysis was completed within 20 minutes. The results showed that 5-HT levels on Day 7 and Day 14 of the culture mediums of IM-treated NSCs were significantly higher than the NSCs in the without IM-treatment NSCs ($p<0.05$; Fig. 21B). Consistent with the results of immunofluorescent assay, we further found that the production of 5-HT on Day 7 and Day 14 of the IM-treated NSCs were significantly diminished by the treatment with BDNF siRNA, Bcl-2 siRNA, or the MAPK inhibitor-U0126 ($p<0.05$; Figs. 21A and B). In contrast to the inhibitory effects of Bcl-2 siRNA, the concentrations of 5-F1T in the siRNA control-treated group were higher than those in the Bcl-2 siRNA-treated and the non-IM-treated groups ($p<0.05$; Figs. 21A and B). Indeed, these results show that the cascades of BDNF/MAPK/ERK pathwayBc1-2 are involved in the serotningeric differentiation process and functional production of neurotransmitters in IM-treated NSCs.

In this example, the immunofluorescence demonstrated that the percentages of serotonin (5-HT)-positive neurons in IM-treated differentiating NSCs were significantly increased with Day 7 IM treatment, but dramatically diminished in Day 7 IM-treated NSCs with the treatment of BDNF siRNA, U0126, or Bcl-2 siRNA (Fig. 20). Furthermore, the microdialysis and HPLC-ED results showed that the functional release of 5-HT in differentiating neurons derived from IM-treated NSCs were concomitantly observed (Fig. 21). In addition, the functional production of 5-HT induced by IM could be effectively inhibited by the treatment with BDNF siRNA, U0126, or Bcl-2 siRNA (Fig. 21). These results indicated that the activation of the BDNF/MAPK/ERK pathway/Bcl-2 cascades play a crucial role in the modulation of functional serotoninergic differentiation in NSCs.

In conclusion, the results of the example proved that the up-regulated BDNF expression and activated cascades of BDNF/MAPK/ERK pathway/Bcl-2 by antidepressant administration in NSCs played a critical role in neuroprotection, inhibition of LPS-induced inflammatory process, facilitation of neural differentiation, and promotion of the functional-release of serotonin in NSCs-derived neurons. In addition to the current understanding concerning the mechanism of IM, *in vitro* culture of NSCs provided an effective system to further elucidate the underlying mechanisms of neurogenesis, neuroprotection, and neurodifferentiation. Moreover, the example supported that *in vitro* monitoring model could be considered a valuable tool to discover new mechanisms and screen candidate response genes in drug research.

**Example 10: Placenta-derived multipotent stem cells induced to differentiate into insulin-positive cells**

Materials and Methods

**Placenta-derived multipotent stem cells (PDMSCs) isolation.**

This example followed the tenets of the declaration of Helsinki and informed consent was obtained from the donor patients. The tissues of human term placenta were dissected and digested by collagenase P (Roche) with Hepes-buffered saline for 7 h at 37 ˚C. The isolation procedure of placenta-derived multipotent stem cells (PDMSCs) isolation was followed as in the previous protocol (Chiou S.H., et al., Biochem. Biophys. Res. Commun., Volume: 326, (2005), pp. 578--585) The dissociated cells obtained from human placenta were negative for CD45 and glycophorin-A after depletion of $CD45^+$ and glycophorin-$A^+$ cells by micromagnetic beads (MACS). These cells were then plated in human fibronectin (FN)-coated (5 ng/ml, Sigma) 96-well plates. Expansion medium consisted of Dulbecco's modified Eagle's medium with 1 g/l of glucose (DMEM-LG, Gibco) and 10% fetal bovine serum (FBS; Gibco) supplemented with 10 ng/ml bFGF, 10 ng/ml EGF, 10 ng/ml PDGF-BB (R&D), 100 units/ml penicillin, and 1000 $\mu$g/ml streptomycin, and 2 mM L -glutamine (Gibco).

**Identification of cell phenotypic markers by FACS.**

PDMCs of passage 5 were used for phenotypic marker identification by flow cytometry. $10^5$ cells were resuspended in 100 $\mu$l PBS and incubated with primary antibodies (anti-human CD13, CD29, CD34, CD44, CD45, CD49b, CD51, CD105, CD166, cKit, MHC I and MHC II (Chemicon)) at 4 ˚C for 1 h with 1:100 dilutions. The labeled cells were suspended in 100 $\mu$l PBS with lul goat anti-mouse IgG conjugated with FITC (Chemicon, AP124F) at 4 ˚C for 1 h, then examined with a FACSCalibur apparatus (Becton-Dickinson).

**Induced PDMSCs differentiated into insulin-positive cells.**

PDMSCs ($10^5$ cells) were then washed twice with HBS solution, then placed on 10 cm and cultured with serum-free ITSFn medium (composed: 1:1 of DMEM/F12, 0.6% Glucose, 25 $\mu$g/ml Insulin, 100 $\mu$g/ml Transferrin, 20 nM Progesterone, 60 $\mu$M Putrescine, 30 nM Selenium chloride, 2 mM Glutamine, 3 mM Sodium bicarbonate, 5 mM Herpes

buffer, 2 μg/ml Heparin, 20 ng/ml EGF, 20 ng/ml b-FGF and 20 ng/ml HGF (PerproTech, Israel). The medium was changed twice and sub-cultured once with the ratio of 1:3 a week.

**Reverse-transcription PCR and SYBR green quantitative-PCR.**

The primers used in the present example were listed in Table 3.

| Gene (GeneBank Code) | Primer sequence | Product size (bp) | Denaturing temperature | Annealing temperature | Extension temperature | Cycles (times) |
|---|---|---|---|---|---|---|
| SOX17 (NM_ 022454) | (f): 5'-GACGACCAGAGCCAGACC-3'<br>(r): 5'-CGCCTCGCCCTTCACC-3' | 114 | 95 | 58 | 72 | 30 |
| Glucagons (NM_ 002054) | (f):5'-GAATACCAAGAGGAACAGGAATAAC-3'<br>(r): 5'-CTTTCACCAGCCAAGCAATG-3' | 140 | 95 | 52 | 72 | 30 |
| Pdx1 (NM 000209) | (f): 5'-AGGAGGAGGACAAGAAGCG-3'<br>(r): 5'-TCTCGGGCGGCAACG-3' | 166 | 95 | 58 | 72 | 30 |
| Somatostatin (NM_ 001048) | (f): 5'-CGCTGTCCATCGTCCTG-3'<br>(r): 5'-GGGCATCATTCTCCGTCTG-3' | 168 | 95 | 54 | 72 | 30 |
| FOXA2 (NM 021784) | (f): 5'-ACTCCATCCGCCACTCG-3'<br>(r): 5'-AGGTAGCAGCCGTTCTCG-3' | 133 | 95 | 54 | 72 | 30 |
| Inslin (NM 000207) | (f): 5'-GGAACGAGGCTTCTTCTACAC-3'<br>(r): 5'-ACAATGCCACGCTTCTGC-3' | 144 | 95 | 55 | 72 | 30 |

SYBR-green was used for quantitative PCR (Q-PCR) detection. Briefly, total RNA (1 μg) of each sample was reverse transcribed in 20 μL using 0.5 μg of oligo-dT and 200 U Superscript II RT (Invitrogen). Amplification was carried out in a total volume of 20 μL containing 0.5 μM of each primer, 4 mM $MgCl_2$, 2 μL LightCycler™-FastStart DNA Master SYBR green I (Rouche) and 2 μL of 1:10 diluted cDNA. All PCRs were performed in triplicate. The transcript levels of genes were standardized to the corresponding GADPH level, and for each gene, mRNA levels relative to the highest candidate gene level were estimated in percentages.

**Immunofluorescent staining.**

The sphere bodies aggregated by PDMSCs were embedded in O.C.T. (Sakura Co.) for frozen sectioning. Sections were fixed by ice-cold acetone (50%) for 2 min at 4 ˚C, then blocked with 5% skin milk at room temperature for 2 h. The sections were then incubated in anti-glucagon (1:500, Abcam), anti-somatostatin (1:500, Chemicon), and anti-insulin (1:500, Chemicon) antibodies in skin milk at 37 ˚C for 2 h, then followed with a 2nd Ab incubation (anti-mouse IgG-Ab with Rhodamine-conjugated insulin detection, Jackson115-095-075; anti-rabbit IgG-Ab with FITC-conjugated glucagons detection, Chemicon).

**Measurement of insulin content/secretion.**

SB-PDMSCs were washed three times with PBS, placed in a 12-well dish with RPMI culture medium supplemented with 10% FBS and the glucose concentration adjusted up to 30 mM (the RPMI contained 5 mM glucose originally) then cultivated for 48 h. The supernatant was collected and centrifuged to examine the insulin secretion, and maintained at -80 ˚C before use. The attached cells were treated with cold acid-ethanol (0.1 N hydrochloric acid in absolute ethanol) and kept at 4 ˚C overnight to examine the insulin content by ELISA (Mercodia) test. The clear supernatants were used to investigate the intracellular insulin content and the values obtained were normalized relative to the total protein content (Bio-Rad). The RIN-m5F insulinoma cell line and PDMSCs were used as controls.

**Transplantation into streptozotocin-induced hyperglycemic mice.**

Eight-week-old severe combined immunodeficiency (SCID) mice were treated with streptozotocin (STZ, 200 mg/kg, Sigma) freshly dissolved in 0.025 M tri-sodium citrate 2 hydrate (pH 4.0). $2 \times 10^5$ PDMSCs and differentiated SB-PDMSCs were injected into the subcapsule of the left kidney of SCID mice following the previously protocol (Soria B., Differentiation, Volume: 68, (2001), pp. 205-219). Blood sampling from the retro-orbital plexus was performed per every two days and measured by using a OneTouch® SureStep (LifeScan Inc.).

**Statistical analysis.**

Statistical analysis was performed by using the one-way or two-way ANOVA test followed by Tukey's test. A $p < 0.05$ was considered to be statistically significant.

By using serum free-pancreatic selection medium (DMEM+EGF+bFGF+HGF+ITS) for 4 weeks of culture, a monolayer appeared, and spindle-like PDMSCs gradually formed 3D spheroid-bodies (SB-PDMSCs; Fig. 22B). The real-time RT-PCR result demonstrated that the mRNA expression levels of endodermal-lineage genes (Sox17 and Foxa2) and a pancreatic development-related gene (Pdxl) were significantly activated in PDMSCs for 3 weeks and then gradually decreased in the fourth week cultures (Figure 22A). The mRNA expression of mature pancreas-related genes (insulin, glucagon, and somatostatin) was significantly up-regulated in the differentiating SB-PDMSCs (*$p<0.05$), but was not observed in undifferentiated PDMSCs in 4-week culture. To further evaluate the protein expression of insulin in these spheroid bodies derived from PDMSCs (SB-PDMSCs; Figure 22B), immunofluorescent assay was used. The results showed that the numbers of the insulin-positive cells were gradually increased in SB-PDMSCs for 4 weeks in pancreatic selection medium culture (Figures 22C and E). The pancreatic islet markers of insulin exhibited a high positive rate and percentage of immune reactivity in the spheroid body of aggregated SB-PDMSCs by immunofluorescent staining (Figures 22C and E). Moreover, both of signals of insulin (red fluorescence) and glucagon (green fluorescence) co-expressed in SB-PDMSCs (Figure 22D). In contrast, the levels of insulin protein in PDMSCs (undifferentiated type) were lower and stably expressed in 4-week culture (Figure 22E).

*In vivo* transplantation test confirmed that SB-PDMSCs could proliferate and form gland-like tissues (Figures 23A and B), differentiate into insulin- and glucagon-positive cells (Figure 23C), and stably restore normoglycemia in STZ-pretreated nude mice (Figure 23D).

The example demonstrated PDMSCs possess the potential to functionally secrete insulin *in vitro* and effectively control blood glucose levels *in vivo.* Furthermore, the microdialysis system of the present invention as show in Figure 10 could make real-time, continuously, and functionally monitor the insulin released from in vitro PDMSCs, SB-PDMSCs, insulin-positive cells, or pancreatic tissues in the different induction condition or drugs stimulation, including pancreatic-induction medium or diabetic drugs.

While the invention has been described and exemplified in sufficient detail for those skilled in this art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The embryos, animals, and processes and methods for producing them are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

## Claims

**1.** A microdialysis system comprising:

(i) a microdialysis apparatus including (a) a microdialysis probe formed to be inserted in a tissue or a transplanted tissue of an animal, the probe defining a probe channel having a perfusate inlet and a dialysate outlet and including a dialysis membrane, the dialysis membrane being formed to permit diffusion of fluid into the channel and to separate the probe channel from the surrounding tissue, (b) an input tube connected to the perfusate inlet; (c) an output tube connected to the dialysate outlet; and
(ii) an assistant device for obtaining the dialysate, which is (d) a rotatory or free moving housing for placing the animal or monitoring *in vivo* treatment response and animal behavior or (e) a container for cultivating a cell medium.

**2.** The system according to claim 1, wherein the input tube is linked to a continuous source of perfusate.

**3.** The system according to claim 1, wherein the output tube is linked to an analyzing device or an autosampling system including time-setting of autopump and/or autocollector.

**4.** The system according to claim 3, wherein the analyzing device is a high performance liquid chromatograpgy system.

**5.** The system according to claim 1, wherein the housing is applied to (a) *in vivo* analysis for a free-moving animal, (b) drug screening or testing, (c) multiple time point or real time monitoring, (d) automatic sampling including time-setting of autopump and/or autocollector; or (e) observation of an animal mode or behavior improvement treated by a drug or small molecule.

**6.** The system according to claim 1, wherein the housing further comprising a light to control or interrupt day and night duration.

**7.** The system according to claim 5, wherein the tissue is brain, heart, liver, bile duct, pancreas, stomach, intestine or kidney.

**8.** The system according to claim 1 wherein the cultivating container is applied

(a) *in vitro* analysis, (b) drug screening or testing, (c) multiple time point or real time monitoring or (d) automatic sampling including time-setting of autopump and/or autocollector.

**9.** The system according to claim 1 wherein the container is in a disk form and the cell is a neuronal cell or attached cell.

**10.** The system according to claim 9 wherein the container has a transparent cover for observing inner space, cell morphological change or cell differentiation-regeneration processes and is positioned in a closed environment.

**11.** The system according to claim 1 wherein the container is in a cone form on the bottom of the container and the cell is a suspending cell, beta-islet cell, insulin-producing cell or hepatic cell.

**12.** The system according to claim 3 wherein the analyzing device is an *in vivo* or *in vitro* green fluorescent protein gene (GFP) imaging device for fluorescent or gene-targeting guidance.

**13.** The system according to claim 12, wherein the fluorescent imaging is expressed on a transplanted hippocampus, ventricle, or intraocular cavity by a transplanted cell with GFP or gene-targeting guidance.

**14.** The system according to claim 13 wherein the cell is a neural stem cell.

**15.** The system according to claim 3 wherein the analyzing device is an apparatus for analyzing gene-related or cytokine function.

**16.** An *in vivo* method for screening or analyzing a target comprising: (a) implanting a microdialysis probe into a tissue of a free-moving animal; (b) placing the animal into a rotatory or free moving housing; (c) adding the target along with a perfusate; and (d) determining the efficacy of the target by screening or analyzing a dialysate.

**17.** The method according to claim 16 further comprising transplanting a cell expressing fluorescence on tissue.

**18.** The method according to claim 16, wherein the probe is implanted into a position exhibiting fluorescence.

**19.** The method according to claim 16, wherein the tissue is brain, liver, bile duct, pancreas, stomach, intestine or kidney.

**20.** The method according to claim 16 wherein the target is a drug for treating or preventing neural or pancreatic diseases.

**21.** An *in vitro* method for screening or analyzing a target comprising: (a) cultivating a cell to a medium in a container; (b) inserting a microdialysis probe into the cell medium; (c) adding the target along with a perfusate; and

(d) determining the efficacy of the target by screening or analyzing a dialysate.

**22.** The method according to claim 21, wherein the cell is neural stem cell, beta-islet cell or insulin-producing cell.

**23.** The method according to claim 21, which further comprises observing morphological alteration, development, differentiation, or observable alteration of the cell.

**24.** The method according to claim 21 wherein the target is a drug for treating or preventing neural or pancreatic diseases.

FIGURE 1

(A)

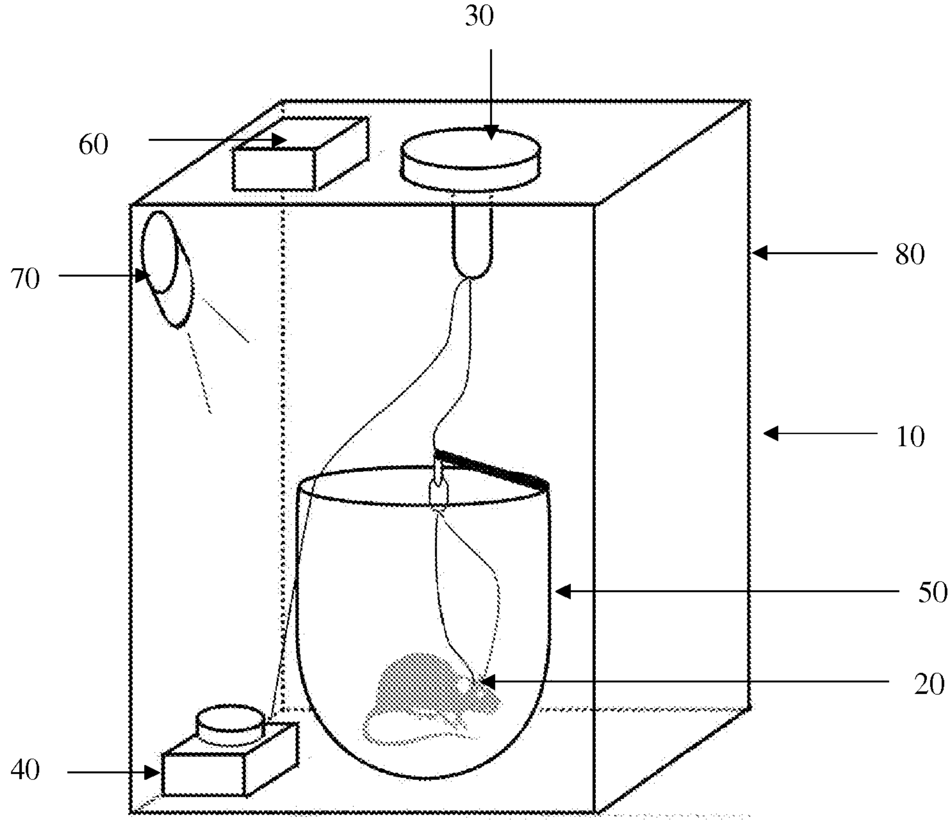


(B)

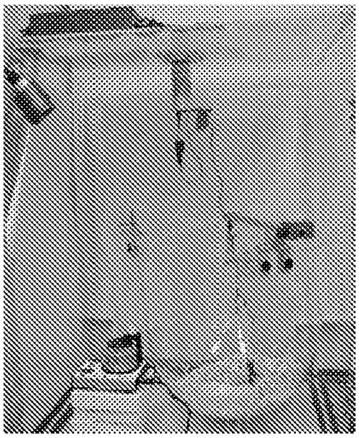

(C)

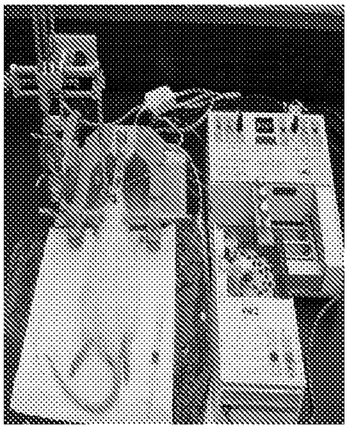

(D)

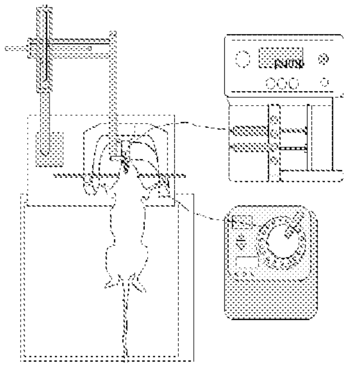

FIGURE 2

(A)

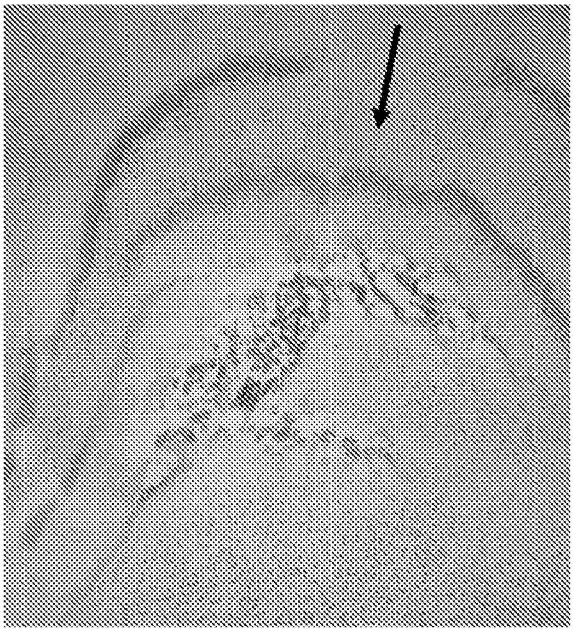

(B)

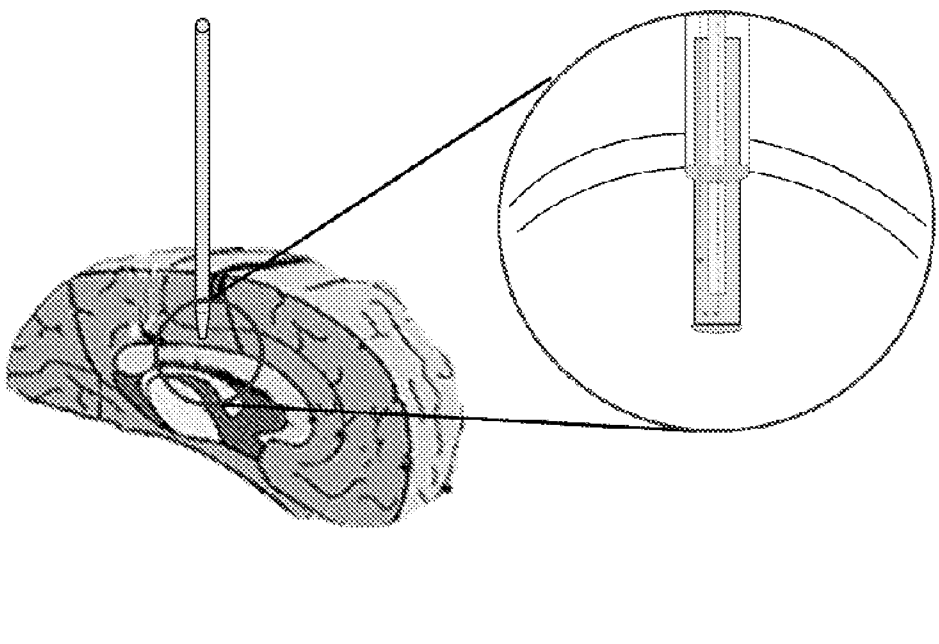

FIGURE 3

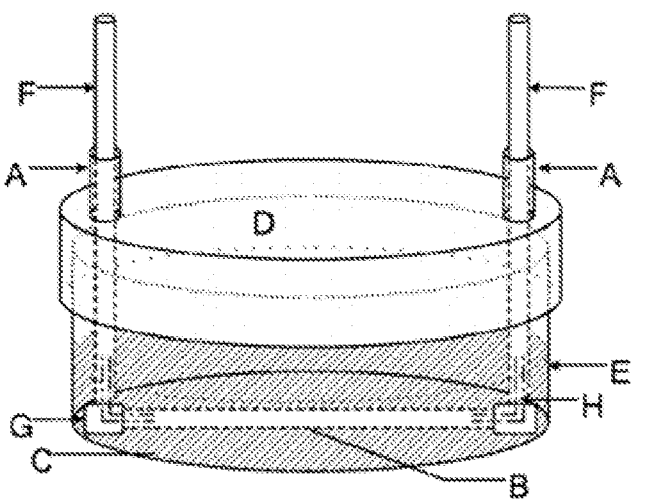

FIGURE 4

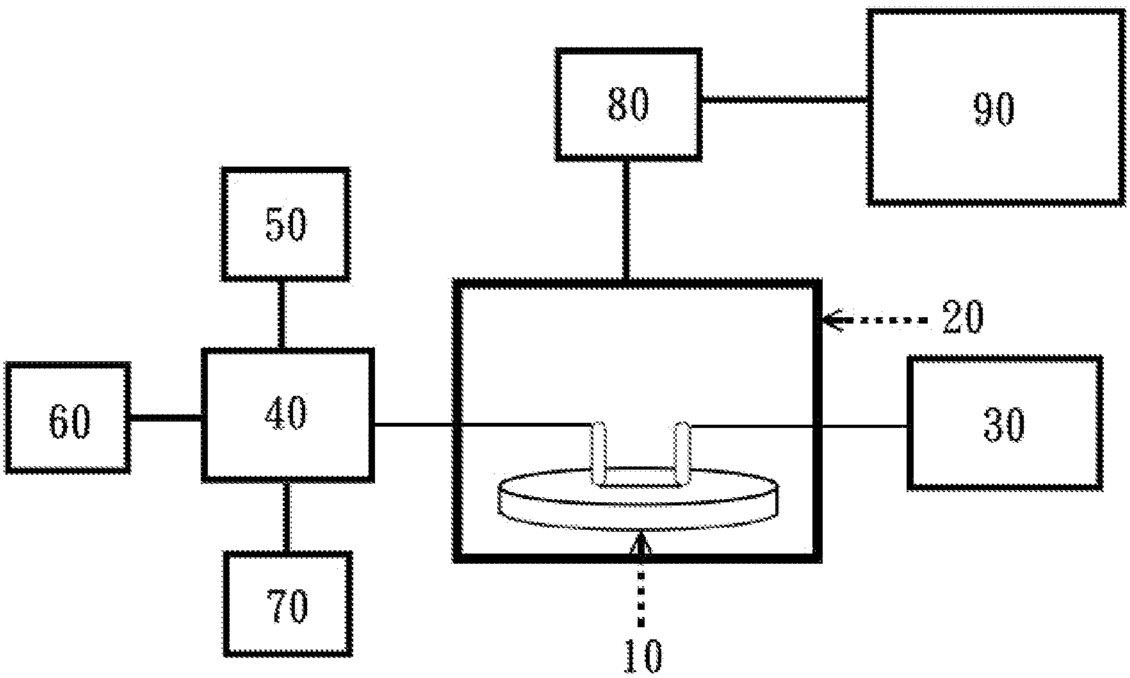

FIGURE 5

(A) (B)

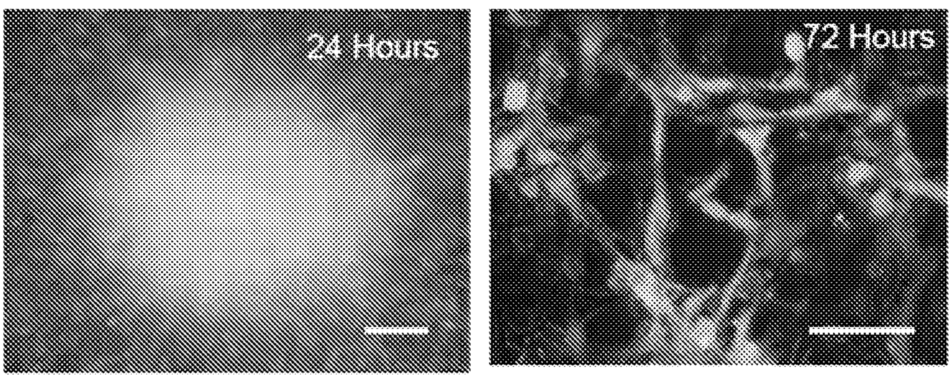

FIGURE 6

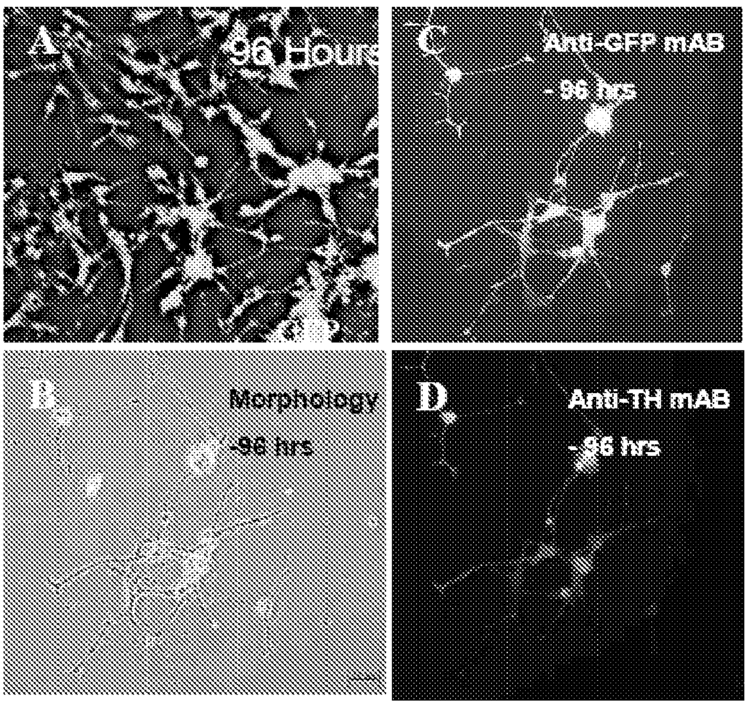
A
96 Hours
C
Anti-GFP mAB
- 96 hrs
B
Morphology
-96 hrs
D
Anti-TH mAB
- 96 hrs

FIGURE 7

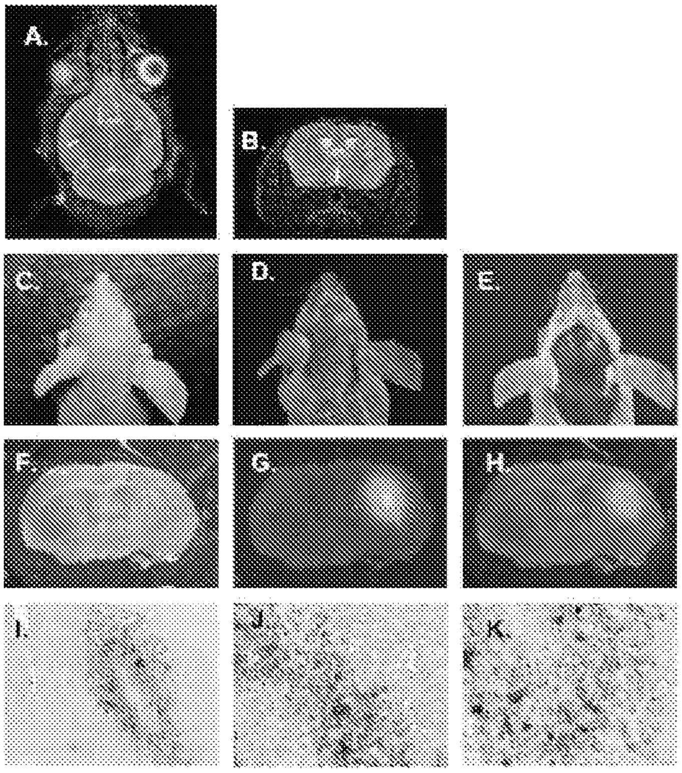


L.

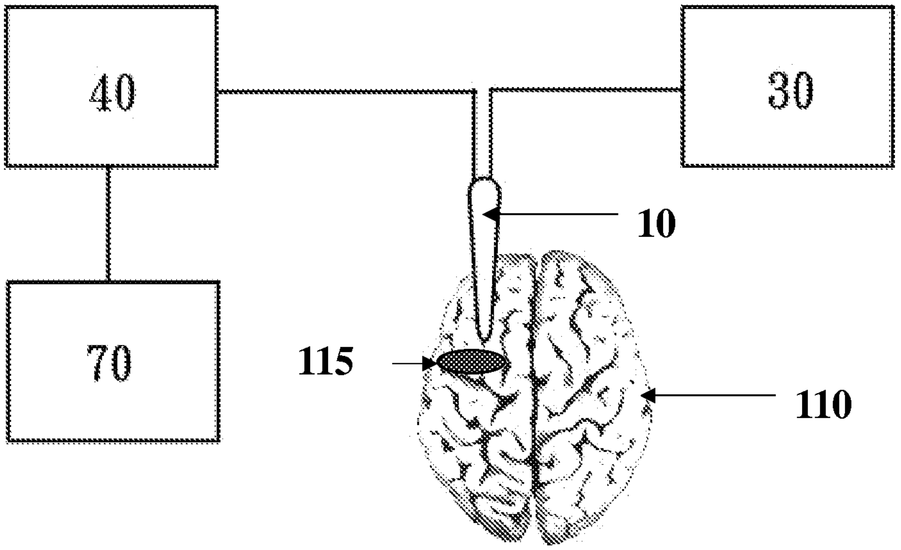

FIGURE 8

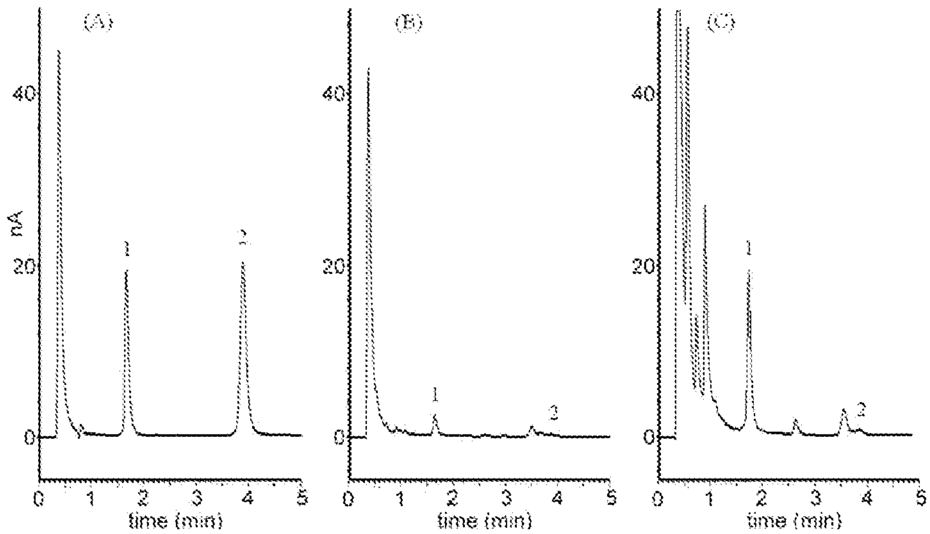

FIGURE 9

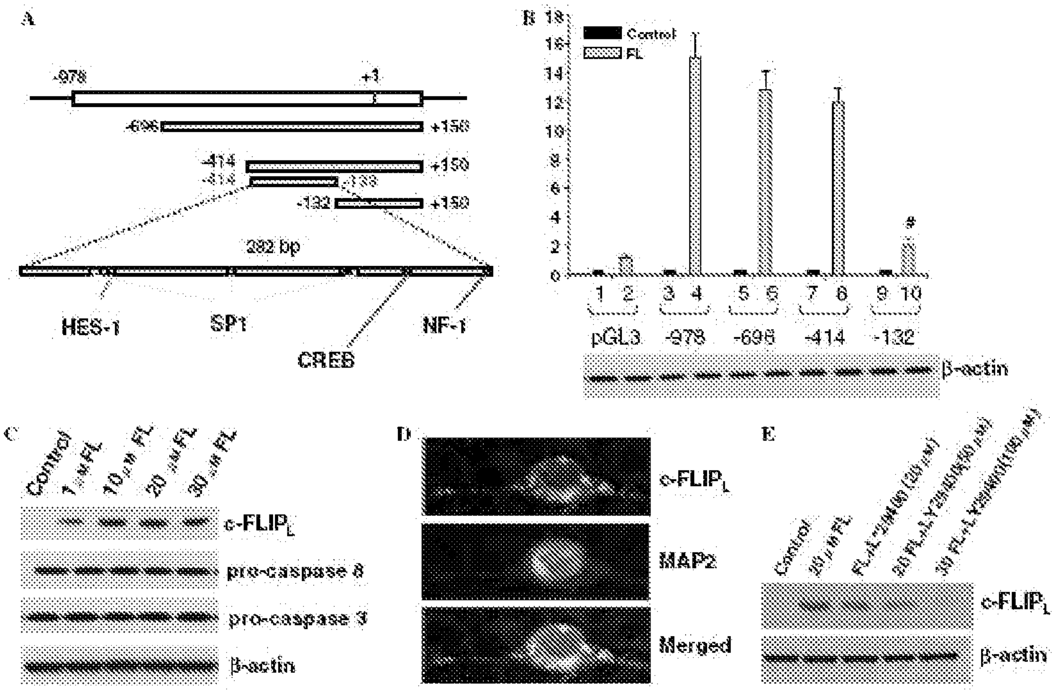
A
-978
+1
-696
+150
-414
+150
-414
-133
-132
+150
282 bp
HES-1
SP1
CREB
NF-1
B
Control
FL
1 2 3 4 5 6 7 8 9 10
pGL3 -978 -696 -414 -132
β-actin
C
Control
1 μM FL
10 μM FL
20 μM FL
30 μM FL
c-FLIP$_L$
pro-caspase 8
pro-caspase 3
β-actin
D
c-FLIP$_L$
MAP2
Merged
E
Control
20 μM FL
c-FLIP$_L$
β-actin

FIGURE 10

(A) (B)

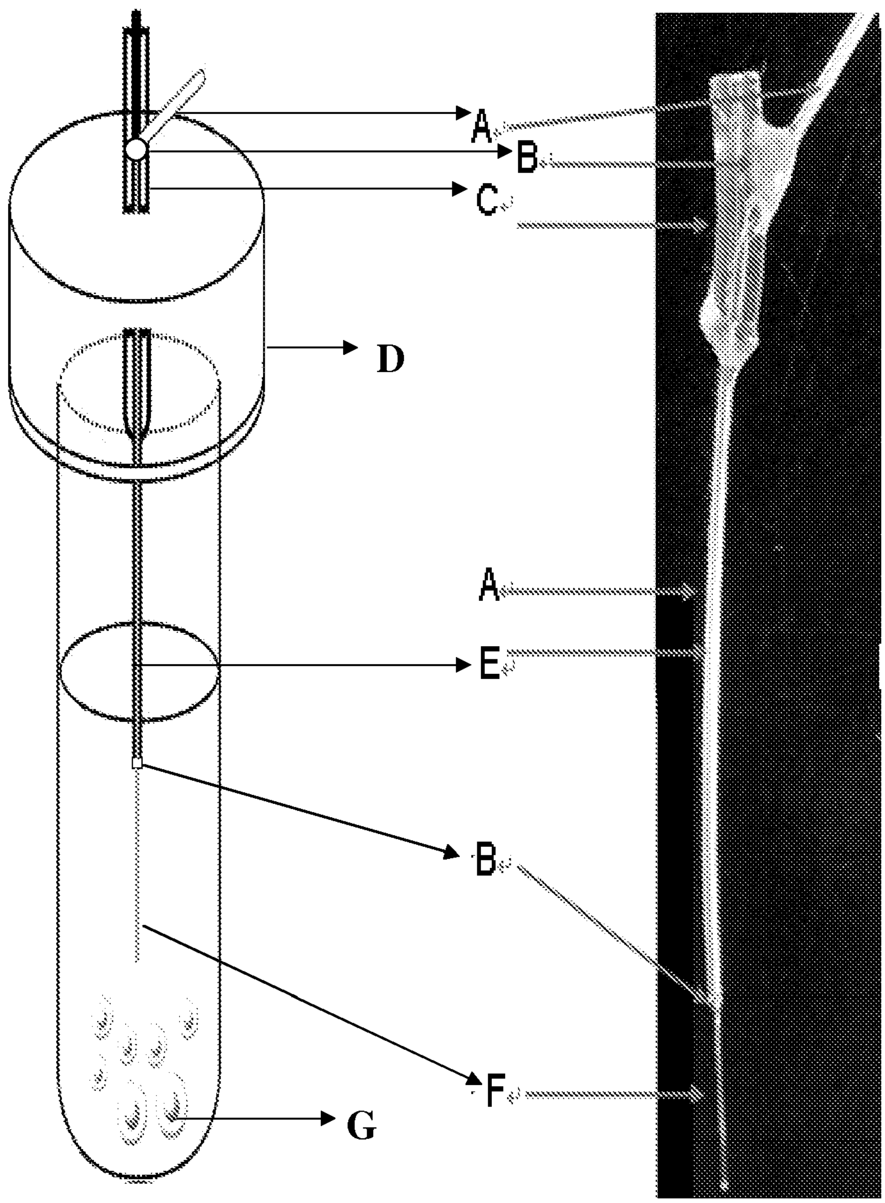

(C)

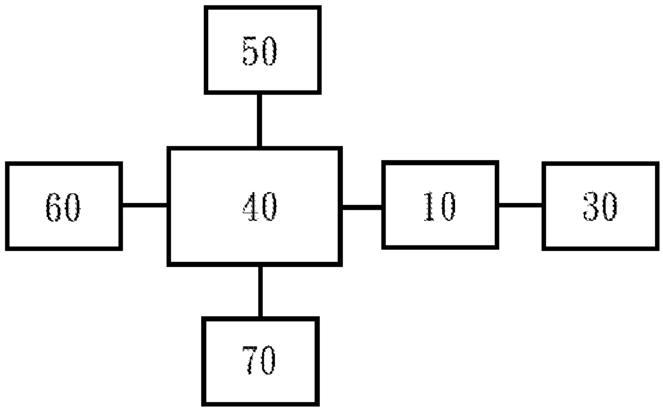
50
60
40
10
30
70

(D)

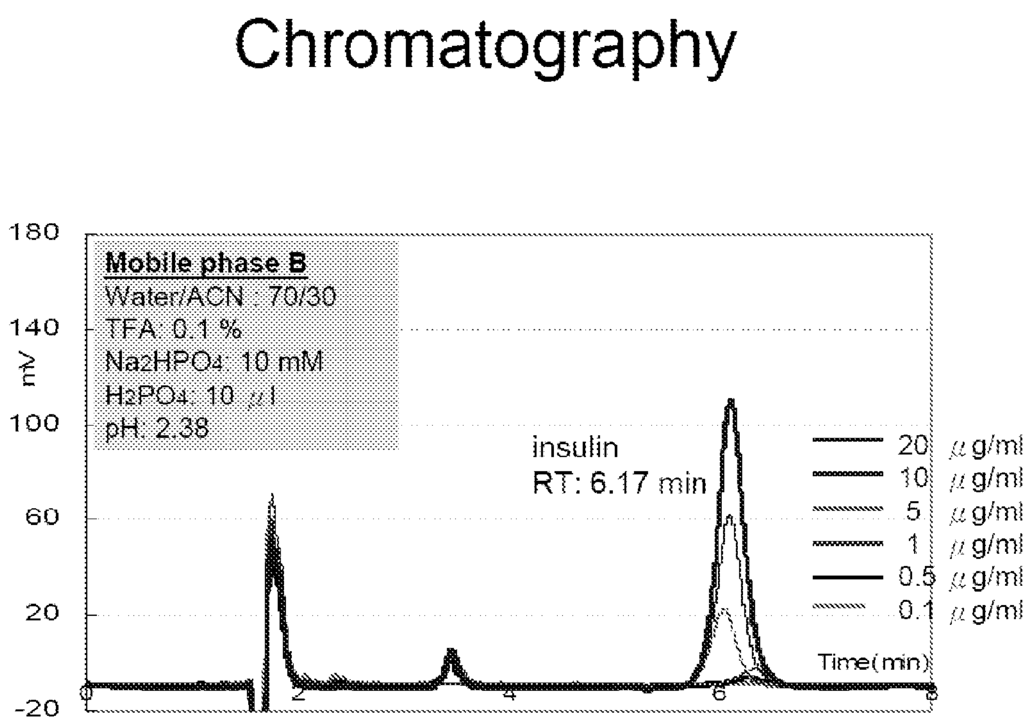
Chromatography
Mobile phase B
Water/ACN : 70/30
TFA: 0.1 %
Na2HPO4: 10 mM
H2PO4: 10 μl
pH: 2.38
insulin
RT: 6.17 min
20 μg/ml
10 μg/ml
5 μg/ml
1 μg/ml
0.5 μg/ml
0.1 μg/ml
mV
Time(min)
180
140
100
60
20
-20
0
2
4
6
8

FIGURE 11

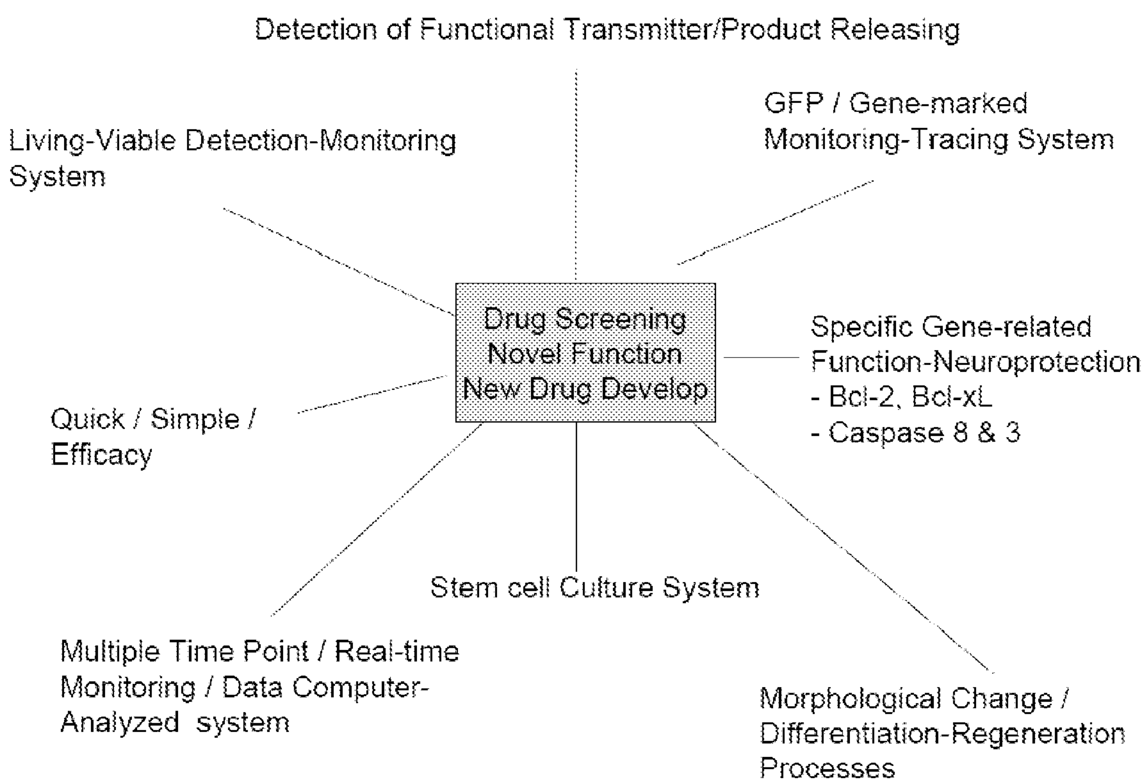
Detection of Functional Transmitter/Product Releasing
Living-Viable Detection-Monitoring System
GFP / Gene-marked Monitoring-Tracing System
Drug Screening
Novel Function
New Drug Develop
Specific Gene-related Function-Neuroprotection
- Bcl-2, Bcl-xL
- Caspase 8 & 3
Quick / Simple / Efficacy
Stem cell Culture System
Multiple Time Point / Real-time Monitoring / Data Computer-Analyzed system
Morphological Change / Differentiation-Regeneration Processes

FIGURE 12

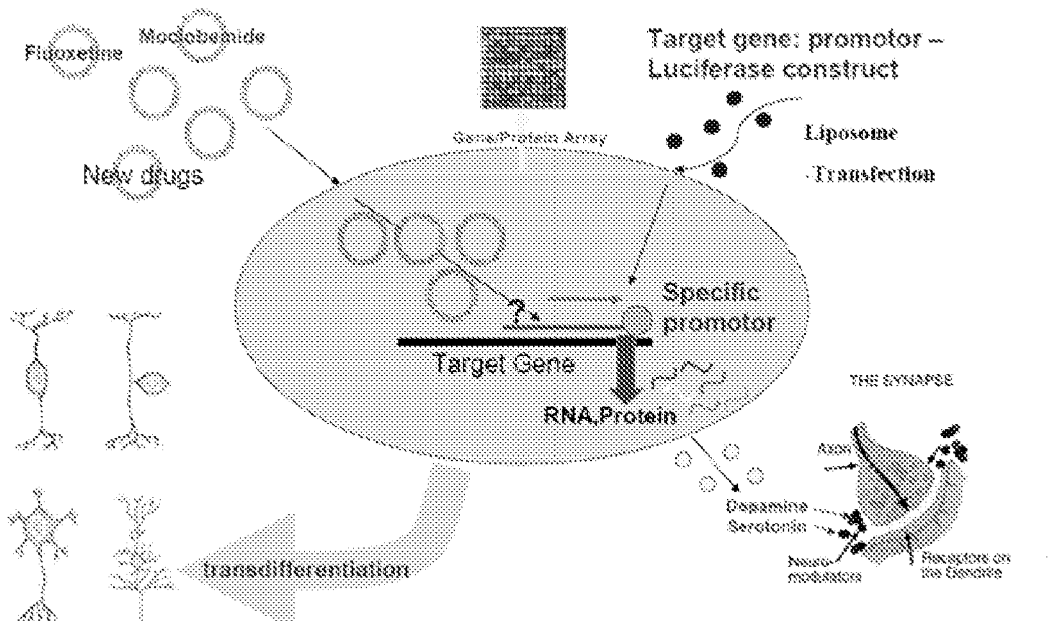
Fluoxetine
Moclobemide
New drugs
Gene/Protein Array
Target gene: promotor –
Luciferase construct
Liposome
Transfection
Specific
promotor
Target Gene
RNA,Protein
THE SYNAPSE
Dopamine
Serotonin
transdifferentiation

FIGURE 13

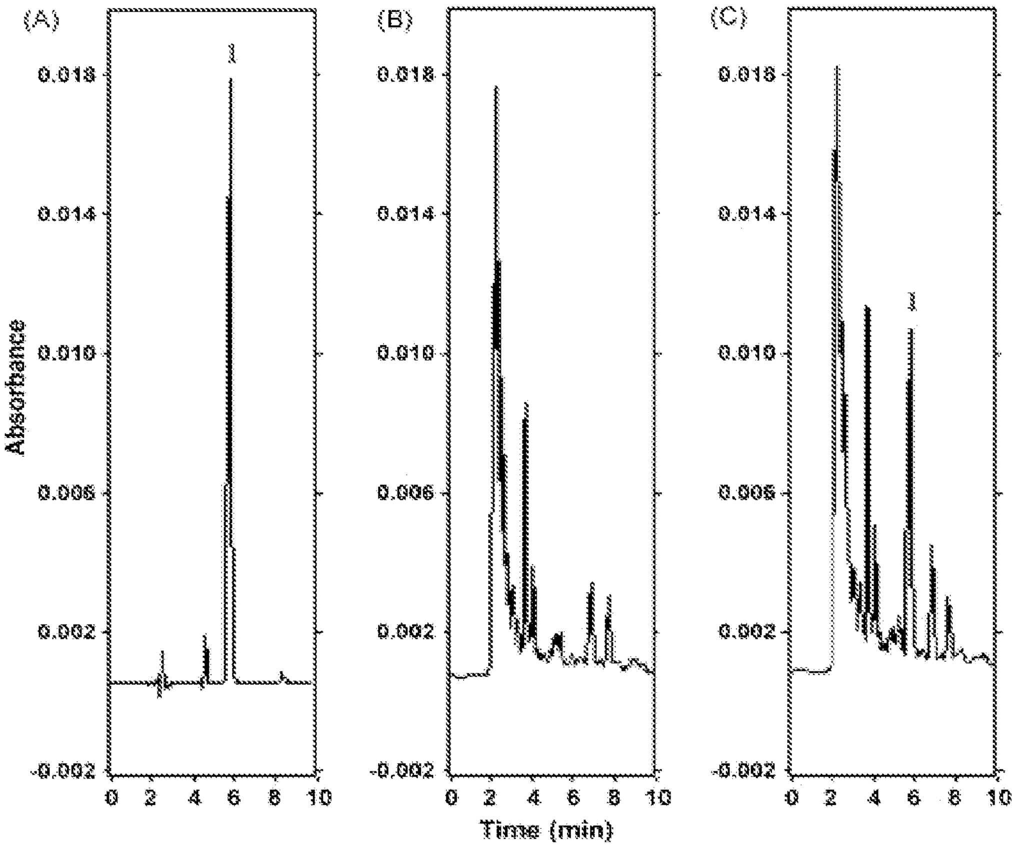
(A)
(B)
(C)
1
1
Absorbance
0.018
0.014
0.010
0.006
0.002
-0.002
0 2 4 6 8 10
Time (min)

FIGURE 14

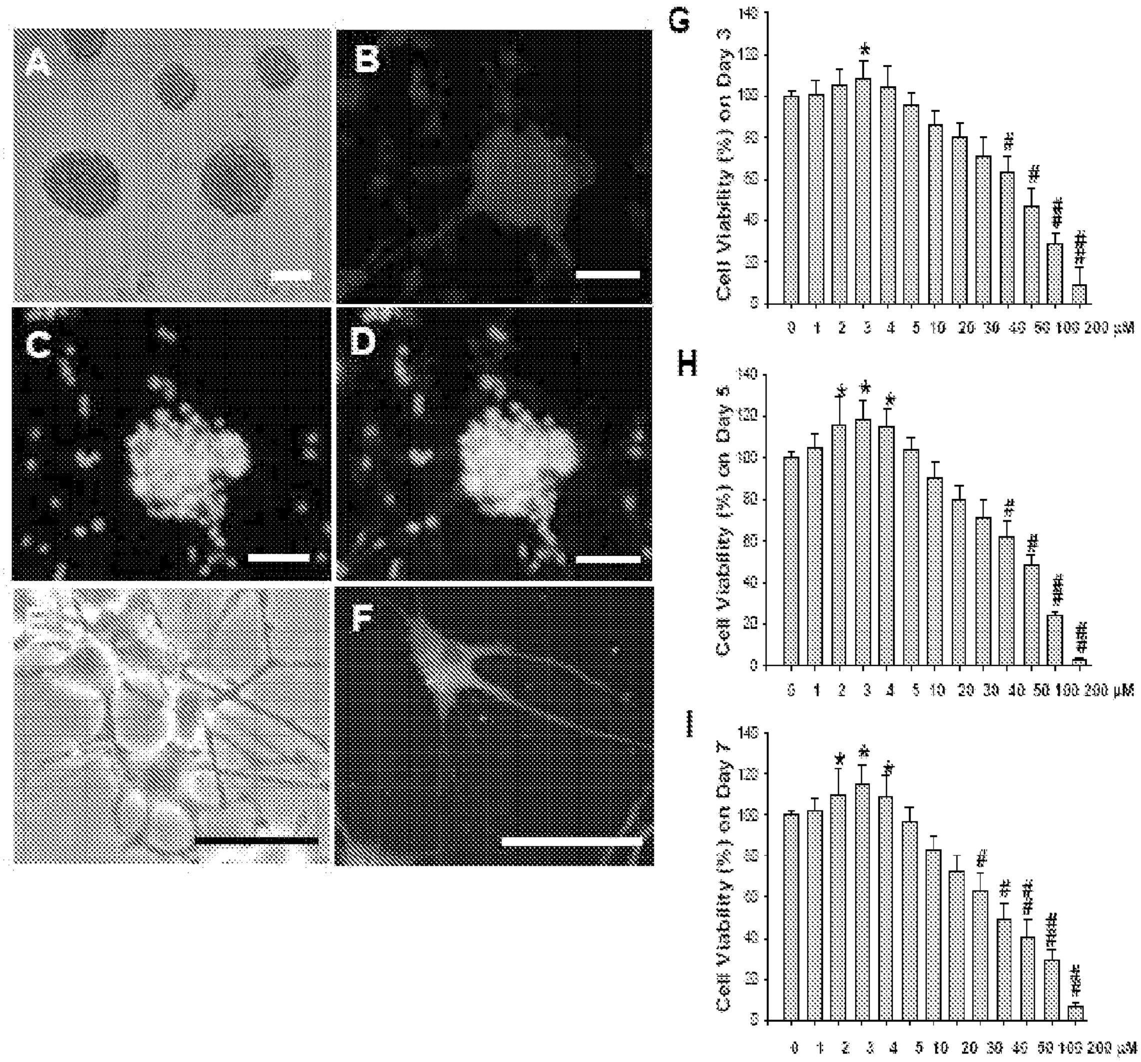
A
B
C
D
F
G
H
I
Cell Viability (%) on Day 3
Cell Viability (%) on Day 5
Cell Viability (%) on Day 7
0 1 2 3 4 5 10 20 30 40 50 100 200 μM

FIGURE 15

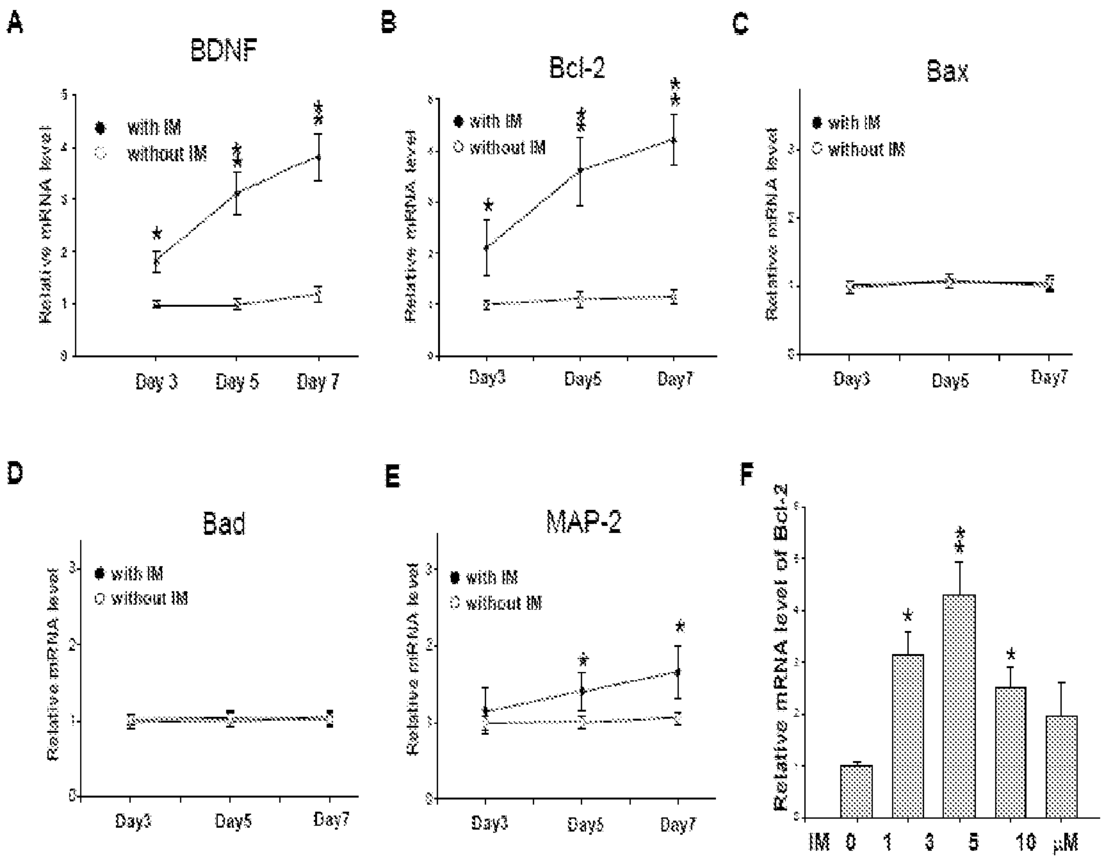
A
BDNF
with IM
without IM
Relative mRNA level
Day 3
Day 5
Day 7
B
Bcl-2
with IM
without IM
Relative mRNA level
Day3
Day5
Day7
C
Bax
with IM
without IM
Relative mRNA level
Day3
Day5
Day7
D
Bad
with IM
without IM
Relative mRNA level
Day3
Day5
Day7
E
MAP-2
with IM
without IM
Relative mRNA level
Day3
Day5
Day7
F
Relative mRNA level of Bcl-2
IM 0 1 3 5 10 μM

FIGURE 16

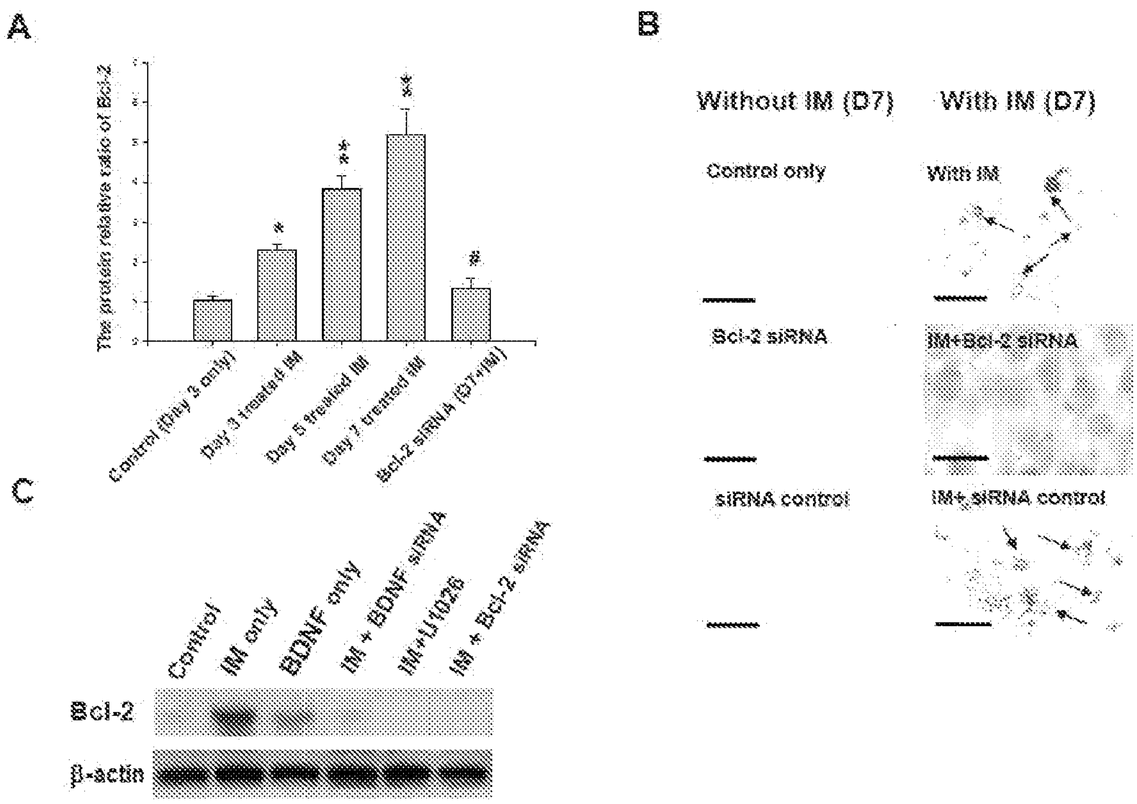
A
The protein relative ratio of Bcl-2
Control (Day 3 only)
Day 3 treated IM
Day 5 treated IM
Day 7 treated IM
Bcl-2 siRNA (D7+IM)
B
Without IM (D7)
With IM (D7)
Control only
With IM
Bcl-2 siRNA
IM+Bcl-2 siRNA
siRNA control
IM+ siRNA control
C
Control
IM only
BDNF only
IM + BDNF siRNA
IM+U1026
IM + Bcl-2 siRNA
Bcl-2
β-actin

FIGURE 17

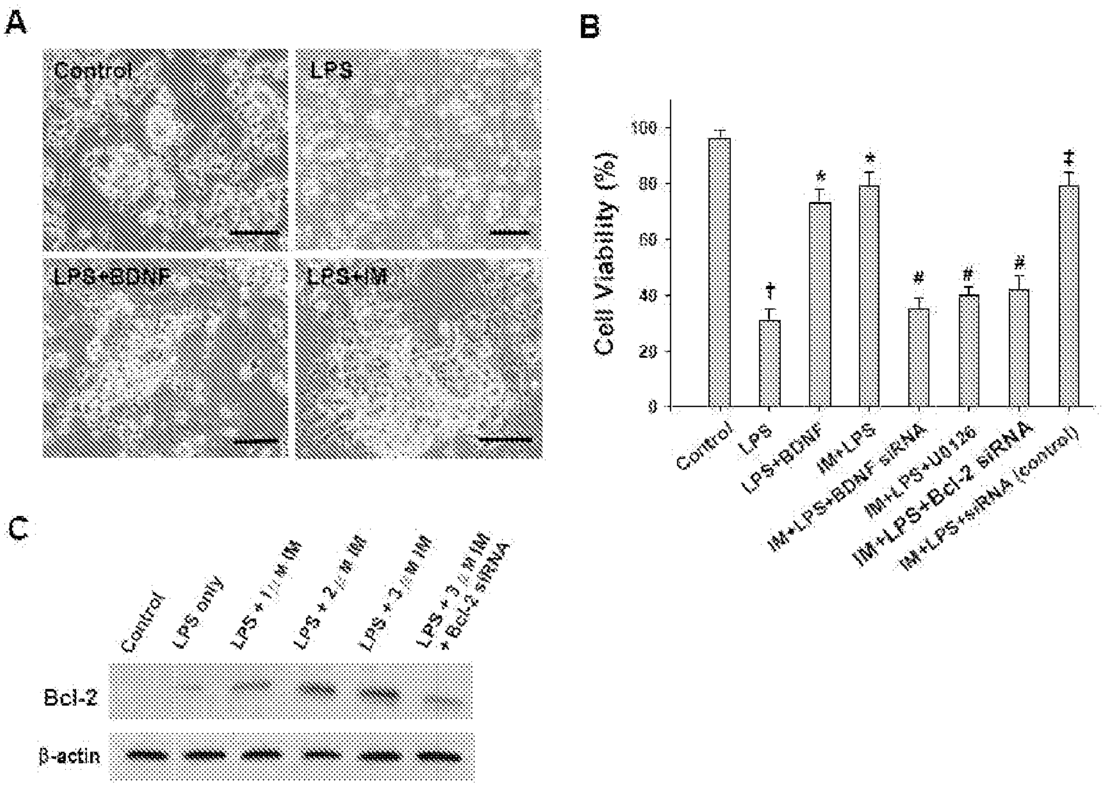
A
Control
LPS
LPS+BDNF
LPS+IM
B
Cell Viability (%)
100
80
60
40
20
0
Control
LPS
LPS+BDNF
IM+LPS
IM+LPS+BDNF siRNA
IM+LPS+U0126
IM+LPS+Bcl-2 siRNA
IM+LPS+siRNA (control)
C
Control
LPS only
+ Bcl-2 siRNA
Bcl-2
β-actin

FIGURE 18

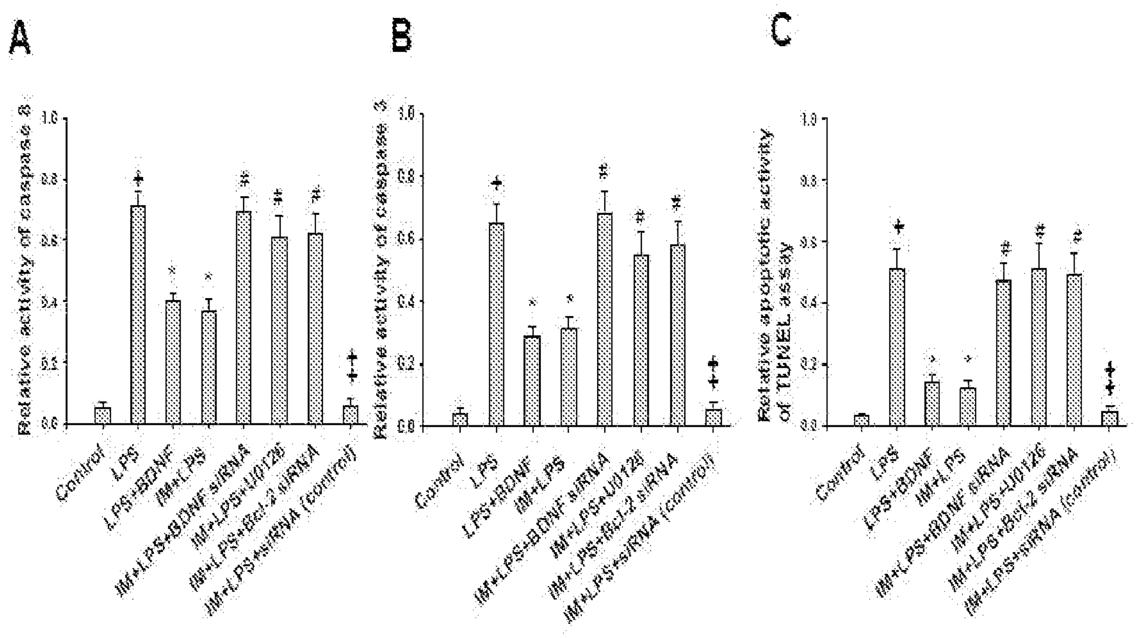
A
B
C
Relative activity of caspase 8
Relative activity of caspase 3
Relative apoptotic activity of TUNEL assay
Control
LPS
LPS+BDNF
IM+LPS
IM+LPS+BDNF siRNA
IM+LPS+U0126
IM+LPS+Bcl-2 siRNA
IM+LPS+siRNA (control)

FIGURE 19

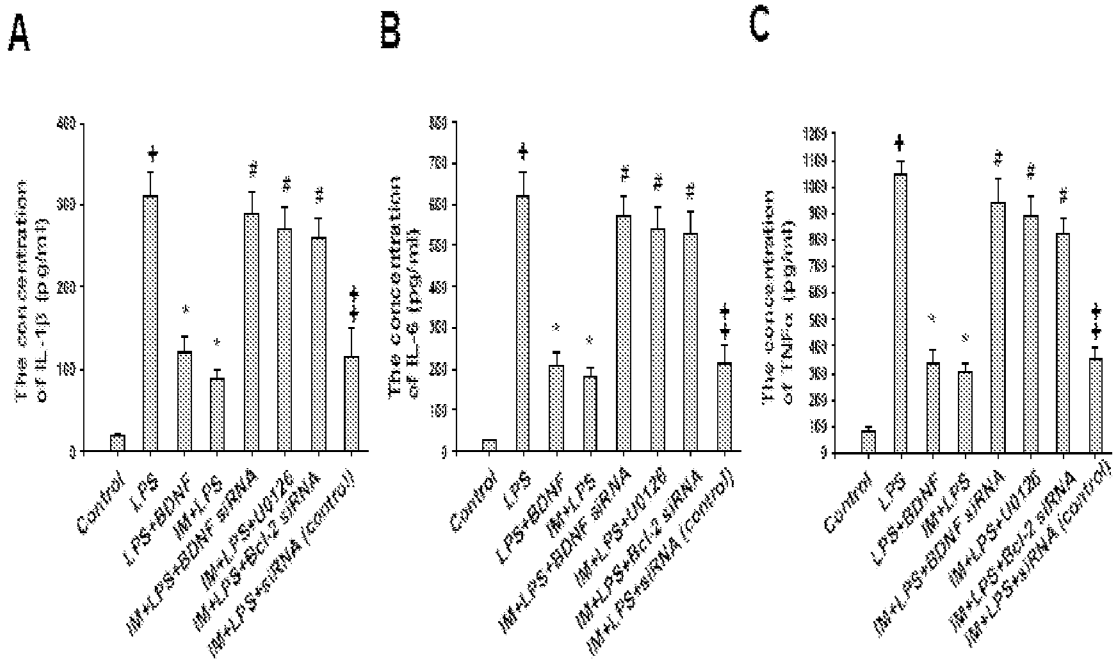
A
B
C
The concentration of IL-1β (pg/ml)
The concentration of IL-6 (pg/ml)
The concentration of TNFα (pg/ml)
Control
LPS
LPS+BDNF
IM+LPS
IM+LPS+BDNF siRNA
IM+LPS+U0126
IM+LPS+Bcl-2 siRNA
IM+LPS+siRNA (control)

FIGURE 20

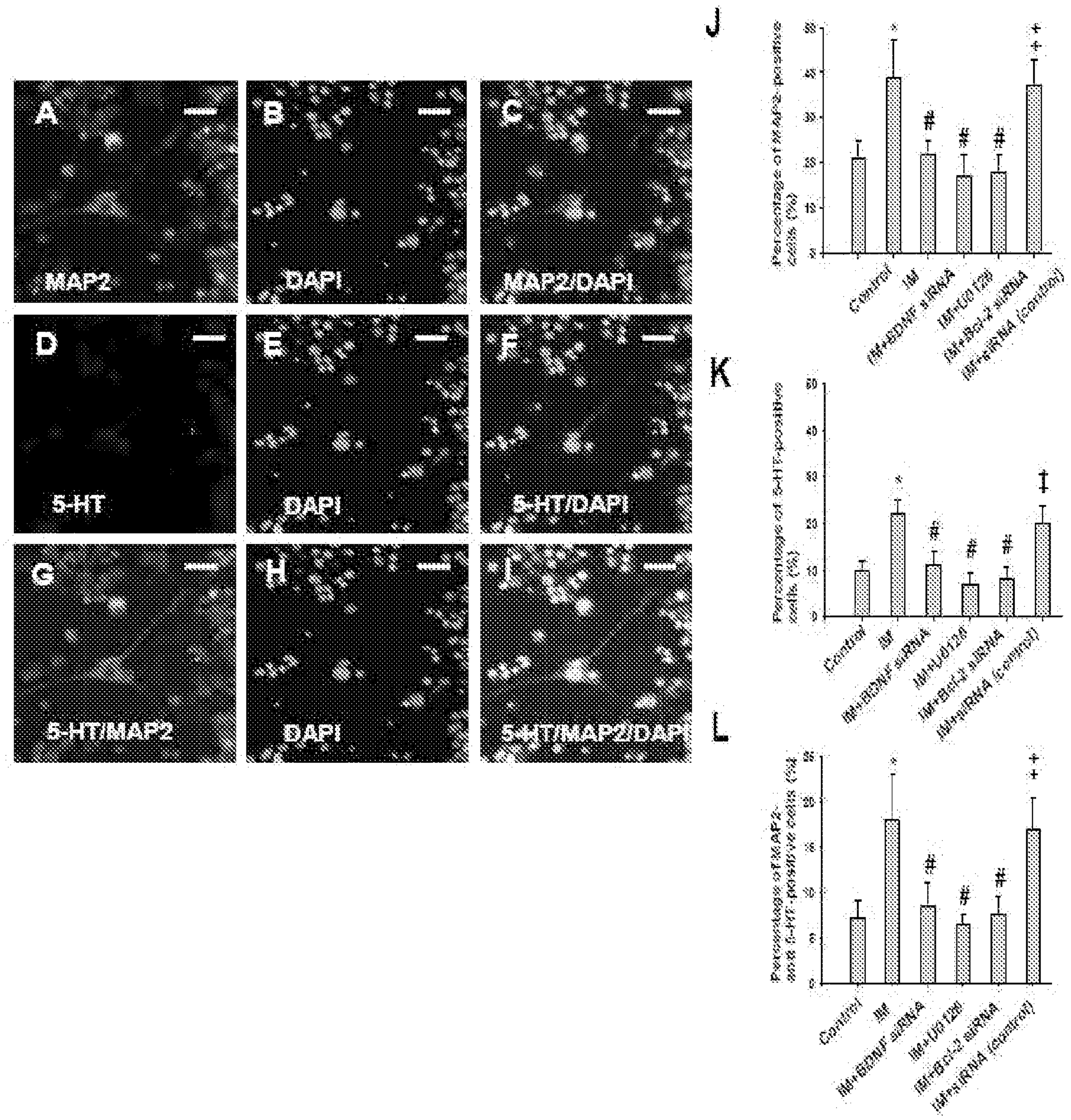
A
MAP2
B
DAPI
C
MAP2/DAPI
D
5-HT
E
DAPI
F
5-HT/DAPI
G
5-HT/MAP2
H
DAPI
I
5-HT/MAP2/DAPI
J
K
L
Control
IM
IM+BDNF siRNA
IM+U0126
IM+Bcl-2 siRNA
IM+siRNA (control)

FIGURE 21

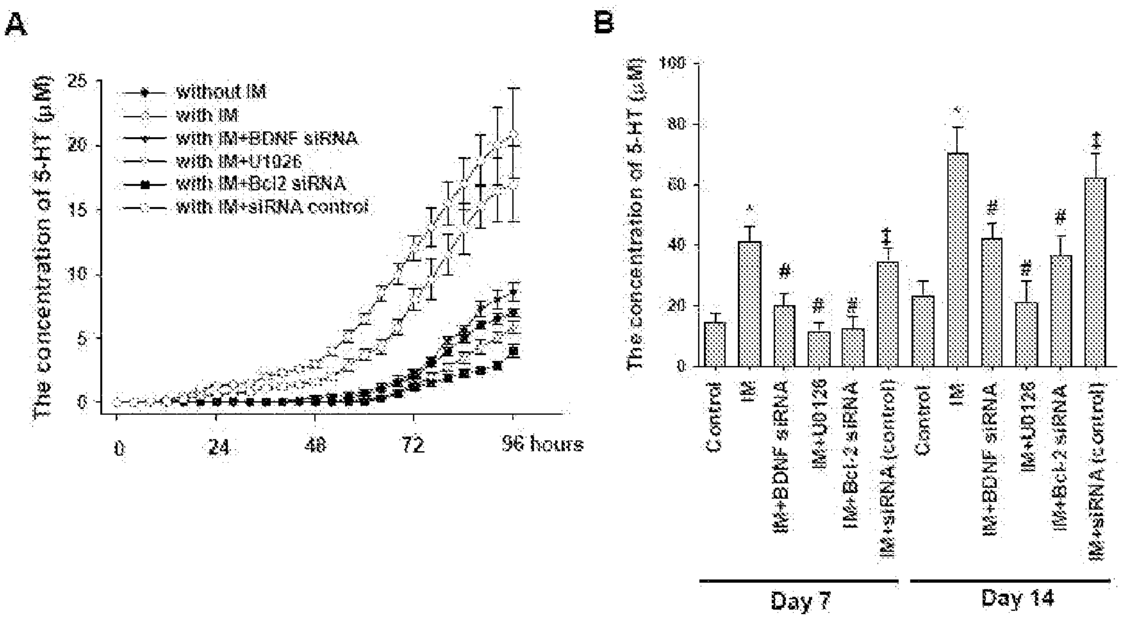
A
The concentration of 5-HT (μM)
25
20
15
10
5
0
0
24
48
72
96 hours
without IM
with IM
with IM+BDNF siRNA
with IM+U1026
with IM+Bcl2 siRNA
with IM+siRNA control
B
The concentration of 5-HT (μM)
100
80
60
40
20
0
Control
IM
IM+BDNF siRNA
IM+U0126
IM+Bcl-2 siRNA
IM+siRNA (control)
Day 7
Day 14

FIGURE 22

A

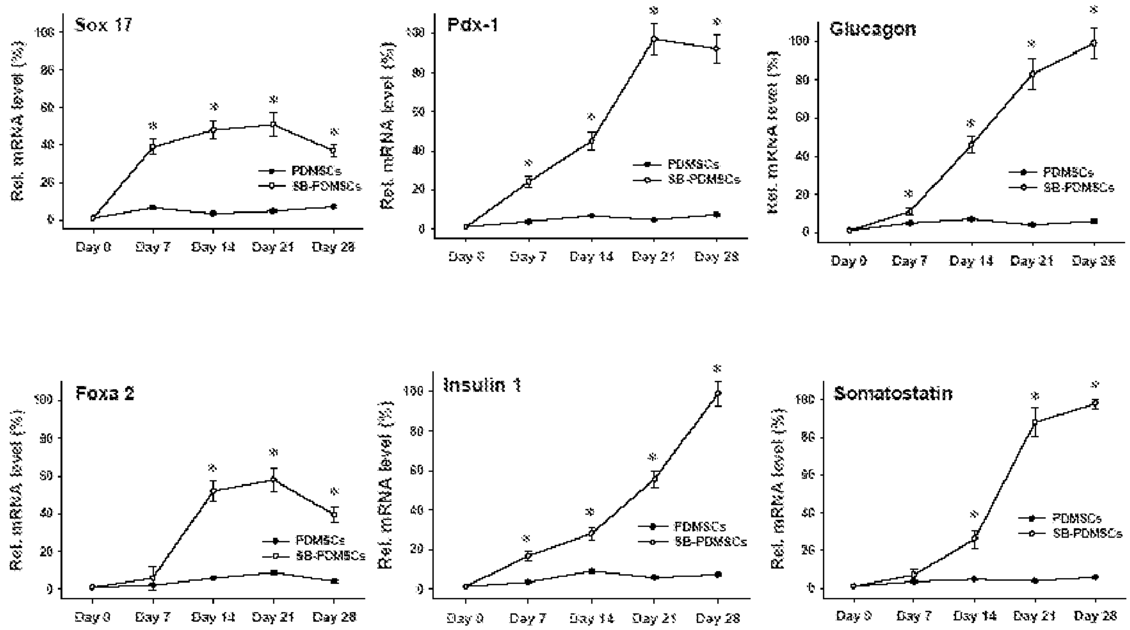
Sox 17
Pdx-1
Glucagon
Foxa 2
Insulin 1
Somatostatin
Rel. mRNA level (%)
PDMSCs
SB-PDMSCs
Day 0
Day 7
Day 14
Day 21
Day 28

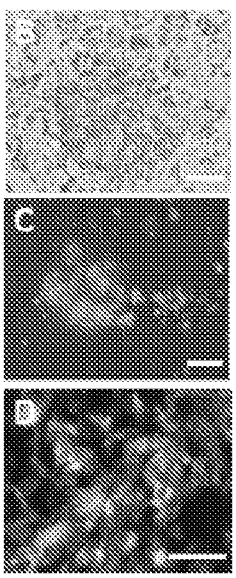

E

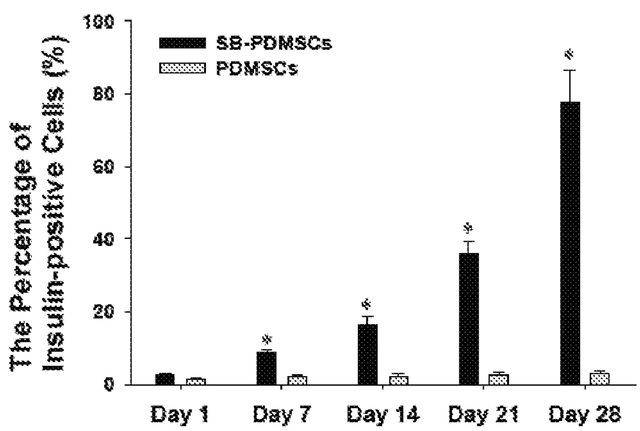
SB-PDMSCs
PDMSCs
The Percentage of Insulin-positive Cells (%)
Day 1
Day 7
Day 14
Day 21
Day 28

FIGURE 23

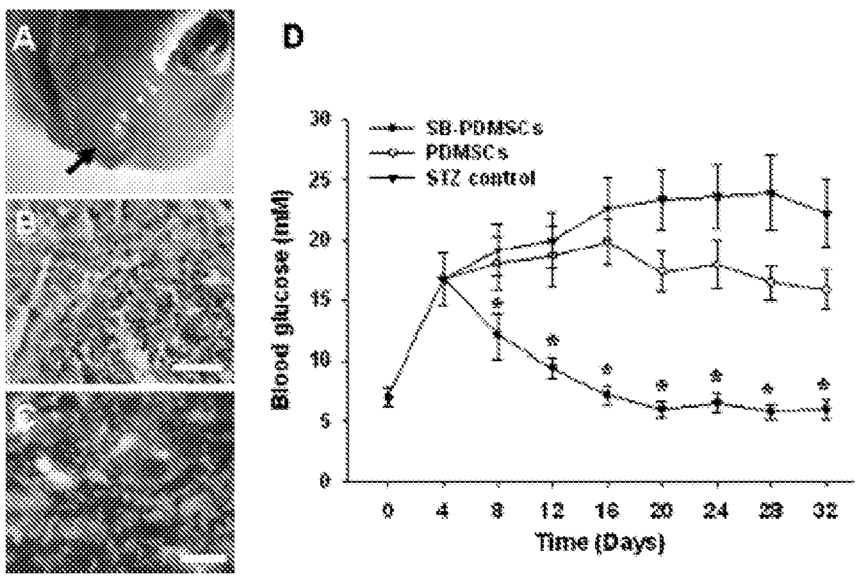
A
B
C
D
SB-PDMSCs
PDMSCs
STZ control
Blood glucose (mM)
Time (Days)
0
5
10
15
20
25
30
0 4 8 12 16 20 24 28 32

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6591126 B
- US 6463312 B
- US 6091976 A
- US 6030358 A
- US 5741284 A
- US 5735832 A
- US 5706806 A
- US 5607390 A

### Non-patent literature cited in the description


- **Shih Hwa Chiou et al.** *Childs Nerv Syst,* 2006, vol. 22, 475-480
- **C.L. Kao et al.** *Mod Pathol,* 2005, vol. 18, 769-778
- **T. Bartke et al.** *Oncogene,* 2001, vol. 20, 571-580
- **Scott D.O. ; Lunte C.E.** *Pharm. Res.,* 1993, vol. 10, 335-342
- **Chiou et al.** *J. Immunol.,* 2001, vol. 167, 4098-4103
- **Cheng et al.** *J. Chromatogr. A.,* 2000, vol. 870, 405-411
- **Franchi et al.** *Biochem. Biophys. Res. Commun.,* 2003, vol. 314, 377-381
- **Yu et al.** *Faseb. J.,* 2005, vol. 19, 1822-1835
- **Chiou S.H. et al.** *Biochem. Biophys. Res. Commun.,* 2005, vol. 326, 578-585
- **Soria B.** *Differentiation,* 2001, vol. 68, 205-219

SEQUENCE LISTING

<110> TAIPEI VETERANS GENERAL HOSPITAL

<120> SYSTEM AND METHODS FOR SCREENING OR ANALYZING TARGETS

<130> 0298-VGH-US

<160> 40

<170> PatentIn version 3.4

<210> 1
<211> 20
<212> DNA
<213> Artificial

<220>
<223> PCR primer

<220>
<221> primer_bind
<222> (1)..(20)

<400> 1
gggccaaaag ggtcatcatc 20

<210> 2
<211> 20
<212> DNA
<213> artificial

<220>
<223> PCR primer

<220>
<221> primer_bind
<222> (1)..(20)

<400> 2
atgaccttgc ccacagcctt 20

<210> 3
<211> 18
<212> DNA
<213> artificial

<220>
<223> PCR primer

<220>
<221> primer_bind
<222> (1)..(18)

<400> 3
ccaaggcagg ttcaagag 18

<210> 4
<211> 19
<212> DNA
<213> artificial

<220>
<223> PCR primer

<220>
<221> primer_bind
<222> (1)..(19)

<400> 4
ccagcagaaa gagaagagg 19

<210> 5
<211> 23
<212> DNA
<213> Artificial

<220>
<223> PCR primer

<220>
<221> primer_bind
<222> (1)..(23)

<400> 5
gggatgactt ctctcgtcgc tac 23

<210> 6
<211> 21
<212> DNA
<213> Artificial

<220>
<223> PCR primer

<220>
<221> primer_bind
<222> (1)..(21)

<400> 6
gttgtccacc aggggtgaca t 21

<210> 7
<211> 23
<212> DNA
<213> Artificial

<220>
<223> PCR primer

<220>
<221> primer_bind
<222> (1)..(23)

<400> 7
gtggttgccc tcttctactt tgc 23

<210> 8
<211> 22
<212> DNA
<213> Artificial

<220>
<223> PCR primer

<220>
<221> primer_bind
<222> (1)..(22)

<400> 8
gaggactcca gccacaaaga tg 22

<210> 9
<211> 25
<212> DNA
<213> Artificial

<220>
<223> PCR primer

<220>
<221> primer_bind
<222> (1)..(25)

<400> 9
gtttacattg ttcaggacct catgg 25

<210> 10
<211> 22
<212> DNA
<213> Artificial

<220>
<223> PCR primer

<220>
<221> primer_bind
<222> (1)..(22)

<400> 10
tcggtaagaa agccagtgtg gt 22

<210> 11
<211> 28
<212> DNA
<213> Artificial

<220>
<223> siRNA

<220>
<221> misc_RNA
<222> (1)..(28)

<400> 11
gatccccgag ctgttggatg aggacctt 28

<210> 12
<211> 18
<212> DNA
<213> Artificial

<220>
<223> siRNA

<220>
<221> misc_RNA
<222> (1)..(18)

<400> 12
agcttaaaaa gagctgtt 18

<210> 13
<211> 21
<212> DNA
<213> Artificial

<220>
<223> siRNA

<220>
<221> misc_RNA
<222> (1)..(21)

<400> 13
gcgcuggaua uaacuucuut t 21

<210> 14
<211> 21
<212> DNA
<213> Artificial

<220>
<223> siRNA

<220>
<221> misc_RNA
<222> (1)..(21)

<400> 14
uucuccgaac gugucacgut t 21

<210> 15
<211> 18
<212> DNA
<213> Artificial

<220>
<223> PCR primer

<400> 15
gacgaccaga gccagacc 18

<210> 16
<211> 16
<212> DNA
<213> Artificial

<220>
<223> PCR primer

<220>
<221> primer_bind
<222> (1)..(16)

<400> 16
cgcctcgccc ttcacc 16

<210> 17
<211> 25
<212> DNA
<213> Artificial

<220>
<223> PCR primer

<220>
<221> primer_bind
<222> (1)..(25)

<400> 17
gaataccaag aggaacagga ataac 25

<210> 18
<211> 20
<212> DNA
<213> Artificial

<220>
<223> PCR primer

<220>
<221> primer_bind
<222> (1)..(20)

<400> 18
ctttcaccag ccaagcaatg 20

<210> 19
<211> 19
<212> DNA
<213> Artificial

<220>
<223> PCR primer

<220>
<221> primer_bind
<222> (1)..(19)

<400> 19
aggaggagga caagcaatg 19

<210> 20
<211> 15
<212> DNA
<213> Artificial

<220>
<223> PCR primer

<220>
<221> primer_bind
<222> (1)..(15)

<400> 20
tctcgggcgg caacg 15

<210> 21
<211> 17
<212> DNA
<213> Artificial

<220>
<223> PCR primer

<220>
<221> primer_bind
<222> (1)..(17)

<400> 21
cgctgtccat cgtcctg 17

<210> 22
<211> 19
<212> DNA
<213> Artificial

<220>
<223> PCR primer

<220>
<221> primer_bind
<222> (1)..(19)

<400> 22
gggcatcatt ctccgtctg 19

<210> 23
<211> 17
<212> DNA
<213> Artificial

<220>
<223> PCR primer

<220>
<221> primer_bind
<222> (1)..(17)

<400> 23
actccatccg ccactcg 17

<210> 24
<211> 18
<212> DNA
<213> Artificial

<220>
<223> PCR primer

<220>
<221> primer_bind
<222> (1)..(18)

<400> 24
aggtagcagc cgttctcg 18

<210> 25
<211> 21
<212> DNA

<213> Artificial

<220>
<223> PCR primer

<220>
<221> primer_bind
<222> (1)..(21)

<400> 25
ggaacgaggc ttcttctaca c 21

<210> 26
<211> 18
<212> DNA
<213> Artificial

<220>
<223> PCR primer

<220>
<221> primer_bind
<222> (1)..(18)

<400> 26
acaatgccac gcttctgc 18

<210> 27
<211> 23
<212> DNA
<213> artificial

<220>
<223> PCR primer

<400> 27
cagcttcata taacccagg gac 23

<210> 28
<211> 24
<212> DNA
<213> artificial

<220>
<223> PCR primer

<400> 28
gctctaggtg gtcattcagg tagg 24

<210> 29
<211> 20
<212> DNA
<213> artificial

<220>
<223> PCR primer

<220>
<221> primer_bind
<222> (1)..(20)

<400> 29
gctgaagtca tccatcaggt 20

<210> 30
<211> 20
<212> DNA
<213> Artificial

<220>
<223> PCR primer

<220>
<221> primer_bind
<222> (1)..(20)

<400> 30
catactgaga tgcaagaatt 20

<210> 31
<211> 21
<212> DNA
<213> Artificial

<220>
<223> PCR primer

<220>
<221> primer_bind
<222> (1)..(21)

<400> 31
agagctgtgg ctaccggtga t 21

<210> 32
<211> 20
<212> DNA
<213> Artificial

<220>
<223> PCR primer

<220>
<221> primer_bind
<222> (1)..(20)

<400> 32
agagggatgg accttgagcg 20

<210> 33
<211> 20
<212> DNA
<213> Artificial

<220>
<223> PCR primer

<220>
<221> primer_bind
<222> (1)..(20)

<400> 33
caccaggtgt gattcaggtg 20

<210> 34
<211> 20
<212> DNA
<213> Artificial

<220>
<223> PCR primer

<220>
<221> primer_bind
<222> (1)..(20)

<400> 34
ccccactgtg ctttgtacct 20

<210> 35
<211> 29
<212> DNA
<213> Artificial

<220>
<223> PCR primer

<220>
<221> primer_bind
<222> (1)..(20)

<400> 35
ggaagatctt aaaaatacaa aaaaaaaaa 29

<210> 36
<211> 29
<212> DNA
<213> Artificial

<220>
<223> PCR primer

<220>
<221> primer_bind
<222> (1)..(29)

<400> 36
ggaagatctg tggctcacgc ctgtaatcc 29

<210> 37
<211> 29
<212> DNA
<213> Artificial

<220>
<223> PCR primer

<220>
<221> primer_bind
<222> (1)..(29)

<400> 37
ggaagatcta gaaagaaaaa aaaaacact 29

<210> 38
<211> 29
<212> DNA
<213> Artificial

<220>
<223> PCR primer

<220>
<221> primer_bind
<222> (1)..(29)

<400> 38
cccaagcttg gtaccgagct cggatccac 29

<210> 39
<211> 21
<212> DNA

<213> Artificial

<220>
<223> siRNA

<220>
<221> misc_RNA
<222> (1)..(21)

<400> 39
ggauccuuca aauaacuuct t 21

<210> 40
<211> 21
<212> DNA
<213> Artificial

<220>
<223> PCR primer

<220>
<221> misc_RNA
<222> (1)..(21)

<400> 40
gaaguuauuu gaaggaucct t 21

SEQUENCE LISTING

<110> TAIPEI VETERANS GENERAL HOSPITAL

<120> SYSTEM AND METHODS FOR SCREENING OR ANALYZING TARGETS

<130> 0298-VGH-US

<160> 40

<170> PatentIn version 3.4

<210> 1
<211> 20
<212> DNA
<213> Artificial

<220>
<223> PCR primer

<220>
<221> primer_bind
<222> (1)..(20)

<400> 1
gggccaaaag ggtcatcatc 20

<210> 2
<211> 20
<212> DNA
<213> artificial

<220>
<223> PCR primer

<220>
<221> primer_bind
<222> (1)..(20)

<400> 2
atgaccttgc ccacagcctt 20

<210> 3
<211> 18
<212> DNA
<213> artificial

<220>
<223> PCR primer

<220>
<221> primer_bind
<222> (1)..(18)

<400> 3
ccaaggcagg ttcaagag 18

<210> 4
<211> 19
<212> DNA
<213> artificial

<220>
<223> PCR primer

<220>
<221> primer_bind
<222> (1)..(19)

<400> 4
ccagcagaaa gagaagagg 19

<210> 5
<211> 23
<212> DNA
<213> Artificial

<220>
<223> PCR primer

<220>
<221> primer_bind
<222> (1)..(23)

<400> 5
gggatgactt ctctcgtcgc tac 23

<210> 6
<211> 21
<212> DNA
<213> Artificial

<220>
<223> PCR primer

<220>
<221> primer_bind
<222> (1)..(21)

<400> 6
gttgtccacc aggggtgaca t 21

<210> 7
<211> 23
<212> DNA
<213> Artificial

<220>
<223> PCR primer

<220>
<221> primer_bind
<222> (1)..(23)

<400> 7
gtggttgccc tcttctactt tgc 23

<210> 8
<211> 22
<212> DNA
<213> Artificial

<220>
<223> PCR primer

<220>
<221> primer_bind
<222> (1)..(22)

<400> 8
gaggactcca gccacaaaga tg 22

<210> 9
<211> 25
<212> DNA
<213> Artificial

<220>
<223> PCR primer

<220>
<221> primer_bind
<222> (1)..(25)

<400> 9
gtttacattg ttcaggacct catgg 25

<210> 10
<211> 22
<212> DNA
<213> Artificial

<220>
<223> PCR primer

<220>
<221> primer_bind
<222> (1)..(22)

<400> 10
tcggtaagaa agccagtgtg gt 22

<210> 11
<211> 28
<212> DNA
<213> Artificial

<220>
<223> siRNA

<220>
<221> misc_RNA
<222> (1)..(28)

<400> 11
gatccccgag ctgttggatg aggacctt 28

<210> 12
<211> 18
<212> DNA
<213> Artificial

<220>
<223> siRNA

<220>
<221> misc_RNA
<222> (1)..(18)

<400> 12
agcttaaaaa gagctgtt 18

<210> 13
<211> 21
<212> DNA
<213> Artificial

<220>
<223> siRNA

<220>
<221> misc_RNA
<222> (1)..(21)

<400> 13
gcgcuggaua uaacuucuut t 21

<210> 14
<211> 21
<212> DNA
<213> Artificial

<220>
<223> siRNA

<220>
<221> misc_RNA
<222> (1)..(21)

<400> 14
uucuccgaac gugucacgut t 21

<210> 15
<211> 18
<212> DNA
<213> Artificial

<220>
<223> PCR primer

<400> 15
gacgaccaga gccagacc 18

<210> 16
<211> 16
<212> DNA
<213> Artificial

<220>
<223> PCR primer

<220>
<221> primer_bind
<222> (1)..(16)

<400> 16
cgcctcgccc ttcacc 16

<210> 17
<211> 25
<212> DNA
<213> Artificial

<220>
<223> PCR primer

<220>
<221> primer_bind
<222> (1)..(25)

<400> 17
gaataccaag aggaacagga ataac 25

<210> 18
<211> 20
<212> DNA
<213> Artificial

<220>
<223> PCR primer

<220>
<221> primer_bind
<222> (1)..(20)

<400> 18
ctttcaccag ccaagcaatg 20

<210> 19
<211> 19
<212> DNA
<213> Artificial

<220>
<223> PCR primer

<220>
<221> primer_bind
<222> (1)..(19)

<400> 19
aggaggagga caagcaatg 19

<210> 20
<211> 15
<212> DNA
<213> Artificial

<220>
<223> PCR primer

<220>
<221> primer_bind
<222> (1)..(15)

<400> 20
tctcgggcgg caacg 15

<210> 21
<211> 17
<212> DNA
<213> Artificial

<220>
<223> PCR primer

<220>
<221> primer_bind
<222> (1)..(17)

<400> 21
cgctgtccat cgtcctg 17

<210> 22
<211> 19
<212> DNA
<213> Artificial

<220>
<223> PCR primer

<220>
<221> primer_bind
<222> (1)..(19)

<400> 22
gggcatcatt ctccgtctg 19

<210> 23
<211> 17
<212> DNA
<213> Artificial

<220>
<223> PCR primer

<220>
<221> primer_bind
<222> (1)..(17)

<400> 23
actccatccg ccactcg 17

<210> 24
<211> 18
<212> DNA
<213> Artificial

<220>
<223> PCR primer

<220>
<221> primer_bind
<222> (1)..(18)

<400> 24
aggtagcagc cgttctcg 18

<210> 25
<211> 21
<212> DNA

<213> Artificial

<220>
<223> PCR primer

<220>
<221> primer_bind
<222> (1)..(21)

<400> 25
ggaacgaggc ttcttctaca c 21

<210> 26
<211> 18
<212> DNA
<213> Artificial

<220>
<223> PCR primer

<220>
<221> primer_bind
<222> (1)..(18)

<400> 26
acaatgccac gcttctgc 18

<210> 27
<211> 23
<212> DNA
<213> artificial

<220>
<223> PCR primer

<400> 27
cagcttcata taacccccagg gac 23

<210> 28
<211> 24
<212> DNA
<213> artificial

<220>
<223> PCR primer

<400> 28
gctctaggtg gtcattcagg tagg 24

<210> 29
<211> 20
<212> DNA
<213> artificial

<220>
<223> PCR primer

<220>
<221> primer_bind
<222> (1)..(20)

<400> 29
gctgaagtca tccatcaggt 20

<210> 30
<211> 20
<212> DNA
<213> Artificial

<220>
<223> PCR primer

<220>
<221> primer_bind
<222> (1)..(20)

<400> 30
catactgaga tgcaagaatt 20

<210> 31
<211> 21
<212> DNA
<213> Artificial

<220>
<223> PCR primer

<220>
<221> primer_bind
<222> (1)..(21)

<400> 31
agagctgtgg ctaccggtga t 21

<210> 32
<211> 20
<212> DNA
<213> Artificial

<220>
<223> PCR primer

<220>
<221> primer_bind
<222> (1)..(20)

<400> 32
agagggatgg accttgagcg 20

<210> 33
<211> 20
<212> DNA
<213> Artificial

<220>
<223> PCR primer

<220>
<221> primer_bind
<222> (1)..(20)

<400> 33
caccaggtgt gattcaggtg 20

<210> 34
<211> 20
<212> DNA
<213> Artificial

<220>
<223> PCR primer

<220>
<221> primer_bind
<222> (1)..(20)

<400> 34
ccccactgtg ctttgtacct 20

<210> 35
<211> 29
<212> DNA
<213> Artificial

<220>
<223> PCR primer

<220>
<221> primer_bind
<222> (1)..(20)

<400> 35
ggaagatctt aaaaatacaa aaaaaaaaa 29

<210> 36
<211> 29
<212> DNA
<213> Artificial

<220>
<223> PCR primer

<220>
<221> primer_bind
<222> (1)..(29)

<400> 36
ggaagatctg tggctcacgc ctgtaatcc 29

<210> 37
<211> 29
<212> DNA
<213> Artificial

<220>
<223> PCR primer

<220>
<221> primer_bind
<222> (1)..(29)

<400> 37
ggaagatcta gaaagaaaaa aaaaacact 29

<210> 38
<211> 29
<212> DNA
<213> Artificial

<220>
<223> PCR primer

<220>
<221> primer_bind
<222> (1)..(29)

<400> 38
cccaagcttg gtaccgagct cggatccac 29

<210> 39
<211> 21
<212> DNA

<213> Artificial

<220>
<223> siRNA

<220>
<221> misc_RNA
<222> (1)..(21)

<400> 39
ggauccuuca aauaacuuct t 21

<210> 40
<211> 21
<212> DNA
<213> Artificial

<220>
<223> PCR primer

<220>
<221> misc_RNA
<222> (1)..(21)

<400> 40
gaaguuauuu gaaggaucct t 21